(19) Europäisches Patentamt
European Patent Office
Office européen des brevets

(11) **EP 3 126 513 B1**

(12) **FASCICULE DE BREVET EUROPEEN**

(45) Date de publication et mention de la délivrance du brevet:
**22.05.2019 Bulletin 2019/21**

(21) Numéro de dépôt: **15713488.3**

(22) Date de dépôt: **31.03.2015**

(51) Int Cl.:
***C12Q 1/6883*** (2018.01)

(86) Numéro de dépôt international:
**PCT/EP2015/057119**

(87) Numéro de publication internationale:
**WO 2015/150426 (08.10.2015 Gazette 2015/40)**

(54) **PROCEDES D'EVALUATION DES EFFETS DELETERES DE L'URINE SUR LA PEAU D'ENFANT**

VERFAHREN ZUR BEWERTUNG DER SCHÄDLICHEN WIRKUNGEN VON URIN AUF DIE HAUT VON KINDERN

METHOD FOR EVALUATING THE HARMFUL EFFECTS OF URINE ON CHILDREN'S SKIN

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **31.03.2014 FR 1452803**

(43) Date de publication de la demande:
**08.02.2017 Bulletin 2017/06**

(73) Titulaire: **Laboratoires Expanscience**
**92048 Paris La Défense Cedex (FR)**

(72) Inventeurs:
• **BAUDOUIN, Caroline**
**78120 Rambouillet (FR)**
• **MSIKA, Philippe**
**F-78000 Versailles (FR)**

(74) Mandataire: **Regimbeau**
**20, rue de Chazelles**
**75847 Paris Cedex 17 (FR)**

(56) Documents cités:
WO-A1-2014/009566      FR-A1- 2 938 193
FR-A1- 2 993 282      US-A1- 2011 020 857

• ARNAUD DEGOUY ET AL: "Baby care product development: Artificial urine in vitro assay is useful for cosmetic product assessment", TOXICOLOGY IN VITRO, vol. 28, no. 1, 11 juillet 2013 (2013-07-11), pages 3-7, XP055141393, ISSN: 0887-2333, DOI: 10.1016/j.tiv.2013.06.022

• MASAKAZU KATOH ET AL: "Assessment of human epidermal model LabCyte EPI-MODEL for in vitro skin irritation testing according to European Centre for the Validation of Alternative Methods (ECVAM)-validated protocol", THE JOURNAL OF TOXICOLOGICAL SCIENCES, vol. 34, no. 3, 1 janvier 2009 (2009-01-01), pages 327-334, XP055141415, ISSN: 0388-1350, DOI: 10.2131/jts.34.327

• SLIVKA SANDRA R ET AL: "Characterization, barrier function, and drug metabolism of an in vitro skin model", THE JOURNAL OF INVESTIGATIVE DERMATOLOGY, NATURE PUBLISHING GROUP, GB, vol. 100, no. 1, 1 janvier 1993 (1993-01-01), pages 40-46, XP002582312, ISSN: 0022-202X, DOI: 10.1111/1523-1747.EP12354098

• ROBINSON M K ET AL: "A STRATEGY FOR SKIN IRRITATION TESTING", AMERICAN JOURNAL OF CONTACT DERMATITIS, SAUNDERS, HARCOURT BRACE JOVANOVICH, PHILADELPHIA, PA, US, vol. 13, no. 1, 1 mars 2002 (2002-03-01), pages 21-29, XP009020639, ISSN: 1046-199X, DOI: 10.1053/AJCD.2002.30471

• RUNEMAN ET AL: "Skin interaction with absorbent hygiene products", CLINICS IN DERMATOLOGY, J.B. LIPPINCOTT, PHILADELPHIA, PA, US, vol. 26, no. 1, 15 février 2008 (2008-02-15), pages 45-51, XP022482744, ISSN: 0738-081X, DOI: 10.1016/J.CLINDERMATOL.2007.10.002

**Description**

La peau est un ensemble de cellules et de macromolécules regroupées sous forme d'un tissu résistant et souple, recouvrant la totalité du corps. Elle est formée de deux couches jointes : l'épiderme et le derme auxquelles on peut associer les tissus sous-cutanés.

L'épiderme dont le principal rôle est la protection du corps constitue la couche la plus superficielle de la peau et assure l'imperméabilité de la peau et sa résistance. On peut identifier dans celui-ci quatre couches cellulaires distinctes, une couche basale (*stratum basalis*), une couche épineuse (*stratum spinosum*), une couche granuleuse (*stratum granulosum*), et une couche cornée (*stratum corneum*). Si différents types cellulaires coexistent dans l'épiderme, les kératinocytes sont largement majoritaires (90 %). Leur activité caractéristique est la synthèse des kératines, des protéines fibreuses et insolubles dans l'eau qui représentent 95 % des protéines totales de l'épiderme.

La peau a pour fonction principale d'établir une barrière de protection contre les atteintes de l'environnement tout en permettant certains échanges entre le milieu intérieur et le milieu extérieur. La fonction de barrière est assurée avant tout par la couche cornée, laquelle rend la peau imperméable et hydrophobe, protégeant ainsi le derme d'une entrée massive d'eau. C'est également la couche cornée qui résiste aux agressions chimiques. Elle se compose de cellules, appelées cornéocytes, mortes et sans noyau, mais remplies de kératines et d'autres produits comme les lipides, les acides gras et les céramides. Les cornéocytes sont reliés par des jonctions serrées particulières, les cornéodesmosomes, formant une couche compacte dont la cohésion est encore renforcée par un ciment lipidique. Sous la couche granuleuse, les jonctions serrées dans la couche granuleuse participent aussi de la fonction de barrière de la peau (voir par exemple, Hogan et al., J Allergy, 2012 : 901940, 2012).

Au quotidien, la peau doit faire face à des attaques diverses. Elle est exposée, par exemple, à des agents chimiques comme le savon ainsi qu'à des stress physiques comme la friction avec les vêtements et l'exposition au soleil. L'épiderme et les annexes épidermiques doivent donc se renouveler constamment pour garder la peau en bon état. Ce sont les cellules souches qui rendent ces processus de maintien et de réparation possibles. Plus particulièrement, la capacité régénérative de l'épiderme est conférée par des cellules souches adultes qui permettent le remplacement régulier des cellules différenciées éliminées lors de la kératinisation. Les cellules couches épidermiques donnent ainsi naissance à des kératinocytes, qui se différencieront pour donner finalement des cornéocytes. Ce processus est en particulier crucial pour la maturation et le maintien de la fonction barrière.

L'adaptation à la vie extra-utérine est un processus qui commence dès la naissance et se poursuit tout au long de la première année de la vie. Les premiers mois de la vie postnatale constituent une période de réorganisation structurelle et fonctionnelle de la peau qui permet une adaptation physiologique à l'environnement extra-utérin. Par exemple, l'immaturité de la peau du nouveau-né est soulignée par la différence de structure et de composition moléculaire du *stratum corneum* par rapport à l'adulte. Celles-ci sont incomplètes et continuent ainsi de se développer durant les 12 premiers mois au moins après la naissance (Chiou et al., Skin Pharmacol Physiol, 17: 57-66, 2004; Nikolovski et al., J Invest Dermatol, 128: 1728-1736, 2008; Stamatas et al., Pediatr Dermatol, 27: 125-131, 2010 ; Telofski et al., Dermatol Res Pract, 2012 : 198789, 2012). Par ailleurs, les résultats de deux études cliniques récentes (Fluhr et al., Br J Dermatol, 166(3) : 483-90, 2012 et Fluhr et al., Br J Dermatol, 2014, doi: 10.1111/bjd.12880) suggèrent que la peau des nourrissons présente une certaine immaturité sur sa capacité à capter l'eau et à réguler les mécanismes qui y sont liés. De plus ces travaux ont montré que la barrière épidermique s'organise structurellement de la naissance à l'âge de 2 ans et n'est donc pas complètement compétente pendant cette période. Ceci contribue à expliquer la fragilité de la peau des nourrissons et jeunes enfants et la susceptibilité de celle-ci aux agressions chimiques, physiques et microbiennes.

De plus, une maturation incomplète de la peau peut avoir des conséquences cliniques importantes. Il est donc important de permettre à la peau de se construire et de se développer correctement et harmonieusement, sans quoi son organisation fonctionnelle et structurelle pourrait en être obérée. À cet égard, il est crucial de préserver la fonction barrière et la capacité de renouvellement de l'épiderme.

L'érythème fessier, ou dermite du siège, est une lésion dermatologique courante chez le bébé porteur de couches (un enfant sur 3), se traduisant par une irritation cutanée pouvant être douloureuse apparaissant là où la couche entre en contact avec la peau. Il résulte le plus souvent de la conjonction de plusieurs facteurs, dont le plus important est le contact prolongé entre la peau du bébé et l'urine. L'urine produit de l'ammoniaque en fermentant et ainsi va contribuer à modifier le pH de la peau qui va alors jouer un rôle délétère important dans la physiopathologie de l'érythème fessier. En outre, les effets de l'urine s'ajoutent à ceux de la transpiration piégée dans les plis de la peau et sous la couche qui font macérer l'épiderme créant un environnement humide et occlusif et augmentant considérablement le pH, l'inflammation et la dégradation de la barrière et ainsi amplifiant les risques de lésions par frottement et donc d'irritations. Dans ce contexte l'urine aggrave ainsi le risque de perte de l'intégrité de la barrière cutanée, ce qui peut conduire à une forme plus grave d'érythème fessier, présentant des plaies ouvertes qui peuvent s'infecter et qui peuvent devoir être soignées par un médecin.

Les produits pharmaceutiques ou cosmétiques disponibles sur le marché contiennent généralement de l'oxyde de zinc, des vitamines (A, D, and D3), ou des combinaisons de ceux-ci. Ces actifs sont incorporés dans une crème ou une pâte à l'eau ou une pommade en les mélangeant avec des bases semi-solides telles que, par exemple, des huiles minérales, de la paraffine, de la lanoline, etc. Ils peuvent être utilisés pour prévenir ou réduire les effets délétères de l'urine, tels que l'érythème fessier. Toutefois, si certains de ces produits permettent d'apporter un soulagement lors des épisodes de légères rougeurs/irritations, ils ne font preuve que d'une efficacité très relative contre les érythèmes fessiers plus importants. Et les produits purement traitants ont souvent une galénique et une concentration d'ingrédients qui les rendent difficilement utilisables à long terme car potentiellement irritants ou allergisants. En outre, ces produits n'ont pour but que d'apaiser la dermite du siège, mais pas de préserver la fonction barrière de la peau ou la capacité de renouvellement de l'épiderme. Il existe donc toujours un besoin, en intégrant les connaissances sur la peau de l'enfant et sur le rôle de facteurs aggravants (comme l'urine et le pH) pour identifier et caractériser des actifs et des formulations améliorés pour prendre en charge au mieux l'érythème fessier.

LEGENDE DES FIGURES

Figure 1 : Production de PGE2, médiateur de l'inflammation lipidique

Figure 2 : Expression du gène PTGS2, médiateur de l'inflammation lipidique

Figure 3 : Expression des gènes IL1alpha et IL8, médiateurs de l'inflammation protéique

Figure 4 : Expression des gènes TRPV1 et SPR, médiateurs de l'inflammation neurogène

Figure 5 : Expression des gènes IVL, SMPD, CSN et CLDN1, marqueurs de la fonction barrière

Figure 6 : Expression des gènes KRT19, ITGB1, ITGB4, ITGA6, BIRC5, Lrig1, marqueurs des cellules souches

DESCRIPTION

Les présents inventeurs ont montré que la peau des enfants, et en particulier celle des nourrissons, est particulièrement sensible à l'action délétère de l'urine. Ils ont en particulier pu identifier des marqueurs biologiques dont l'expression est modifiée en présence d'urine. De tels marqueurs sont particulièrement avantageux parce qu'ils permettent de suivre la réponse de la peau à l'urine.

Les inventeurs ont développé des procédés d'évaluation de l'efficacité *in vitro* de principes actifs et de formulations sur la prévention des effets délétères de l'urine pour la peau d'enfant, utilisant un modèle de peau spécifiquement capable de reproduire les caractéristiques de la peau des enfants, et en particulier celle des enfants très jeunes comme les nourrissons. Les études de l'art antérieur reposaient sur l'utilisation de la peau d'adulte pour analyser la réponse de la peau à l'urine (Degouy et al., Toxicol In Vitro, 28(1) : 3-7. 2014). Cependant, la propriétés de la peau évoluent durant les premières années de la vie (voir par exemple Fluhr et al., Exp Dermatol., 19(6) : 483-492, 2010 ; Fluhr et al., Br J Dermatol, 166(3) : 483-90, 2012 et Fluhr et al., Br J Dermatol., 2014, doi: 10.1111/bjd.12880) et il est peu probable qu'il soit possible de déterminer précisément les effets de l'urine sur la peau des enfants à partir d'échantillons de peau d'adulte.

En revanche, les inventeurs ont mis au point des modèles de peau reconstruite à partir de d'échantillons provenant d'enfant et ont pu tester l'effet de l'urine sur ces modèles. Ils ont ainsi pu observer que l'expression de certains marqueurs biologiques était altérée quand ces modèles de peau reconstruite d'enfant étaient mis en contact avec l'urine. Certains marqueurs, comme les marqueurs de l'inflammation étaient ainsi plus fortement exprimés, tandis que l'expression d'autres, tels que les marqueurs des cellules souches ou ceux de la fonction barrière était diminuée. En revanche, les variations d'expression de ces marqueurs étaient réduites, voire estompées, quand les modèles étaient traités avec des actifs ou des formulations connues pour apaiser l'érythème fessier. Ce résultat souligne la pertinence physiologique de ces marqueurs. L'importance de l'utilisation de modèles de peau reconstruite d'enfants et non pas d'adultes pour isoler de tels marqueurs en est d'ailleurs renforcée.

Par « enfant », on entend selon l'invention un individu dont l'âge est inférieur à 16 ans. Sont ainsi compris dans la catégorie des enfants selon l'invention, les nouveau-nés, dont l'âge est compris entre 0 et 1 mois, les nourrissons, qui ont entre 1 mois et 2 ans, et les enfants proprement dits, qui sont âgés d'au moins 2 ans. Un « nouveau-né », comme on l'entend ici, peut aussi bien être né à terme qu'être prématuré.

Pour lever toute ambiguïté, le terme « enfant » utilisé icisans autre précision doit être entendu dans son acception la plus générale, c'est-à-dire comme se référant à une personne de moins de 16 ans. Un « adulte » au sens de la présente invention et description est une personne qui n'est pas un enfant, autrement dit une personne âgée de plus de 16 ans.

Préférablement, la méthode de l'invention et de la description peut être utilisée quelle que soit l'origine ethnique ou géographique de la peau, ou encore le phototype de celle-ci. Elle peut ainsi être d'origine caucasienne, africaine, asiatique, sud-américaine, mélanésienne ou autre ; elle peut encore présenter un phototype I, II, III, IV, V ou VI, sans que cela n'affecte la méthode. Celle-ci a en effet pour but l'identification de marqueurs biologiques caractérisant n'importe quel type de peau et ne dépendant que de l'âge du donneur.

Les procédés de l'invention et de la description reposent donc sur l'utilisation d'un modèle de peau adapté, reproduisant la peau d'enfant, ainsi que sur l'utilisation de marqueurs biologiques, dont l'expression est affectée par l'urine de manière particulière dans la peau des enfants. Ces procédés permetent ainsi de déterminer de façon précise quels actifs ont un effet avantageux sur la prévention ou le traitement des effets délétères causés par la mise en contact de la peau avec l'urine. Les procédés de l'invention et de la description sont aussi adaptés à l'évaluation de l'activité de formulations. Les inventeurs ont ainsi pu montrer que certaines formulations étaient plus efficaces que d'autres pour prévenir et/ou limiter les effets de l'urine, démontrant ainsi l'utilité de l'approche entreprise.

Il est ici décrit un procédé d'évaluation de l'efficacité *in vitro* d'un actif ou d'une formulation sur la prévention des effets délétères de l'urine sur la peau d'enfant, caractérisé en ce que ledit procédé comprend les étapes suivantes:

a) mettre en contact ledit actif ou ladite formulation avec un modèle de peau reconstruite, ledit modèle étant obtenu à partir d'un échantillon cutané provenant d'un enfant ;
b) exposer à l'urine le modèle de peau reconstruite de l'étape a) ;
c) mesurer le niveau d'expression d'au moins un marqueur biologique dans le modèle de peau de l'étape b) ; et
d) évaluer l'efficacité dudit actif ou de ladite formulation en fonction du niveau de l'étape c).

L'invention concerne en particulier un procédé d'évaluation de l'efficacité *in vitro* d'un actif ou d'une formulation sur la prévention des effets délétères de l'urine sur la peau d'enfant tel que défini dans les revendications.

De préférence, le modèle de peau reconstruite est exposé à l'urine dans l'étape b) en présence de l'actif ou de la formulation. Alternativement, l'actif ou la formulation est éliminée préalablement à l'exposition dudit modèle de peau reconstruite à l'urine lors de ladite étape b).

Par ailleurs, il est ici décrit un procédé d'évaluation de l'efficacité *in vitro* d'un actif ou d'une formulation dans la réduction des effets délétères de l'urine sur la peau d'enfant, caractérisé en ce que ledit procédé comprend les étapes suivantes:

a) exposer à l'urine un modèle de peau reconstruite, ledit modèle étant obtenu à partir d'un échantillon cutané provenant d'un enfant ;
b) mettre en contact ledit actif ou ladite formulation avec le modèle de peau reconstruite de l'étape a) ;
c) mesurer le niveau d'expression d'au moins un marqueur biologique dans le modèle de peau de l'étape b) ; et
d) évaluer l'efficacité dudit actif ou de ladite formulation en fonction du niveau de l'étape c).

L'invention concerne en particulier un procédé d'évaluation de l'efficacité *in vitro* d'un actif ou d'une formulation dans la réduction des effets délétères de l'urine sur la peau d'enfant tel que défini dans les revendications.

L'homme du métier comprendra sans peine que les étapes a) et b) peuvent être réalisées simultanément ou successivement, selon ses besoins. Autrement dit, le modèle de peau reconstruite peut être mis en contact à la fois avec l'urine et l'actif ou la formulation testé. Alternativement, le modèle de peau peut d'abord être exposé à l'urine, puis mis en contact avec l'actif ou la formulation.

Par « l'efficacité d'une formulation ou d'un actif dans la prévention ou la réduction des effets délétères de l'urine sur la peau d'enfant », on entend ici la capacité de la formulation ou de l'actif à annuler ou diminuer lesdits effets délétères sur une peau d'enfant. La prévention s'entend dans le cas présent d'un traitement qui est administré avant le développement des effets délétères de l'urine, tandis que la réduction correspond à un traitement qui est administré une fois que les effets de l'urine sont apparus.

Selon un mode de réalisation plus préféré, le donneur de l'échantillon est plus particulièrement un donneur ayant un âge compris entre 0 et 1 mois, entre 1 mois et 2 ans ou entre 2 ans et 16 ans. Autrement dit, selon ce mode de réalisation, le donneur de l'échantillon est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans. Plus préférentiellement, le donneur de l'échantillon est un nouveau-né ou un nourrisson.

Par exemple, le niveau d'expression dudit marqueur biologique de l'étape a) est comparé à un niveau d'expression de référence.

Il est important de vérifier que les actifs et les formulations des présents procédéssont bien tolérés. Par exemple, certains produits actuellement commercialisés peuvent entrainer des irritations s'ils sont employés régulièrement. Un tel effet ne peut qu'empirer un érythème fessier en développement ou déjà présent.

Il est donc ici décrit un procédé d'évaluation de la tolérance d'un actif ou d'une formulation lorsque la peau d'enfant est exposée à l'urine, ledit procédé comprenant les étapes suivantes :

> a) mettre en contact ledit actif ou ladite formulation avec un modèle de peau reconstruite, ledit modèle étant obtenu à partir d'un échantillon cutané provenant d'un enfant ;
> b) exposer à l'urine en présence de l'actif ou de la formulation le modèle de peau reconstruite de l'étape a) ;
> c) mesurer le niveau d'expression d'au moins un marqueur biologique dans le modèle de peau de l'étape b) ; et
> d) déterminer si ledit agent actif, l'actif ou de la formulation est bien toléré par la peau d'enfant en fonction du niveau de l'étape c).

L'invention concerne en particulier un procédé d'évaluation de la tolérance d'un actif ou d'une formulation sur la peau d'enfant exposée à l'urine tel que défini dans les revendications.

Le procédé peut en outre comporter une comparaison de la viabilité cellulaire dans le modèle de peau reconstruite traité avec l'actif, la composition ou la formulation et dans le modèle de peau reconstruite témoin, c'est-à-dire traité avec l'urine seule. Dans ce cas, l'actif ou la formulation cosmétique est bien toléré par la peau d'enfant si la viabilité cellulaire du modèle de peau reconstruite n'est pas affectée par la présence de l'agent ou de la formulation.

De préférence, le procédé comprend donc une étape supplémentaire de détermination de la viabilité cellulaire dans le modèle de peau reconstruite exposé à l'urine et traité avec l'actif ou la formulation cosmétique, de détermination de la viabilité cellulaire du modèle de peau reconstruite témoin et de comparaison entre les deux.

De nombreux tests pour déterminer la viabilité cellulaire sont disponibles pour l'homme du métier et sont couramment utilisés en cosmétologie. On citera en particulier le test MTT, décrit par exemple dans Mosman et al. (J Immunol Methods, 65(1-2) : 55-63, 1983).

Dans un autre aspect, les procédés de l'invention et de la description permettent d'isoler des formulations ou des actifs ayant un effet dans la prévention des effets délétères de l'urine sur la peau d'enfant. Comme le montrent les exemples expérimentaux, les procédés de l'invention et de la description permettent en particulier de distinguer les actifs ou les formulations selon leur activité pour prévenir l'effet délétère de l'urine sur la peau d'enfant. Les procédés sont donc particulièrement adaptés pour identifier des formulations ou des actifs appropriés à cette peau très spécifique.

Il est donc ici décrit un procédé d'identification d'un actif ou d'une formulation pour la prévention des effets délétères de l'urine sur la peau d'enfant, caractérisé en ce que ledit procédé comprend les étapes suivantes:

> a) mettre en contact un actif ou une formulation candidat avec un modèle de peau reconstruite, ledit modèle étant obtenu à partir d'un échantillon cutané provenant d'un enfant ;
> b) exposer à l'urine le modèle de peau de l'étape a) ;
> c) mesurer le niveau d'expression d'au moins un marqueur biologique dans le modèle de peau de l'étape b) ; et
> d) déterminer si ledit actif ou ladite formulation candidat est une formulation ou un actif pour la prévention des effets délétères de l'urine sur la peau d'enfant en fonction du niveau de l'étape c).

L'invention concerne en particulier un procédé d'identification d'un actif ou d'une formulation pour la prévention des effets délétères de l'urine sur la peau d'enfant tel que défini dans les revendications.

De préférence, le modèle de peau reconstruite est exposé à l'urine dans l'étape b) en présence de l'actif ou de la formulation. Alternativement, l'actif ou la formulation est éliminée préalablement à l'exposition dudit modèle de peau reconstruite à l'urine lors de ladite étape b).

De même, la méthode de l'invention et de la description permet d'isoler des actifs ou des formulations permettant de réduire les effets délétères de l'urine sur la peau d'enfant. Par exemple , cette méthode comprend les étapes suivantes:

> a) exposer à l'urine un modèle de peau reconstruite, ledit modèle étant obtenu à partir d'un échantillon cutané provenant d'un enfant ;
> b) mettre en contact un actif ou une formulation candidat avec le modèle de peau de l'étape a) ;
> c) mesurer le niveau d'expression d'au moins un marqueur biologique dans le modèle de peau de l'étape b) ; et
> d) déterminer si ledit actif ou ladite formulation candidat est une formulation ou un actif pour la réduction des effets délétères de l'urine sur la peau d'enfant en fonction du niveau de l'étape c).

L'invention concerne en particulier un procédé d'identification d'un actif ou d'une formulation pour la réduction des effets délétères de l'urine sur la peau d'enfant tel que défini dans les revendications.

L'homme du métier comprendra sans peine que les étapes a) et b) peuvent être réalisée simultanément ou successivement, selon ses besoins. Autrement dit, le modèle de peau reconstruite peut être mis en contact à la fois avec l'urine et l'actif ou la formulation testé. Alternativement, le modèle de peau peut d'abord être exposé à l'urine, puis mis en contact avec l'actif ou la formulation.

La formulation candidate est une formulation pour la prévention ou la réduction des effets délétères de l'urine sur la peau d'enfant, si ladite formulation candidate permet de moduler l'expression d'au moins un marqueur biologique de l'invention et de la description. Cette modulation peut correspondre, selon les cas, et en particulier selon la nature du marqueur biologique, à une augmentation ou à une diminution de l'expression dudit marqueur. Par exemple, il peut être intéressant d'isoler des formulations minimisant les effets délétères de l'urine sur les marqueurs préférentiellement exprimés dans les cellules souches, ces formulations permettant de préserver la capacité de renouvellement de l'épiderme fragile des enfants. De même, il serait avantageux d'identifier des formulations minimisant les effets délétères de l'urine sur les marqueurs de la barrière chez les enfants, afin de maintenir l'intégrité de la barrière cutanée. Enfin, il pourrait être désirable d'isoler des formulations qui n'induiraient pas les marqueurs de l'inflammation.

De la même façon, l'actif candidat est un actif pour la prévention ou la réduction des effets délétères de l'urine sur la peau d'enfant, si ledit actif candidat permet de moduler l'expression d'au moins un marqueur biologique de l'invention et de la description. Cette modulation peut correspondre, selon les cas, et en particulier selon la nature du marqueur biologique, à une augmentation ou à une diminution de l'expression dudit marqueur.

Par « urine », on entend un liquide formé d'environ 95 % d'eau et d'environ 5 % de matières organiques et de sels minéraux, principalement chlorures, sulfates et phosphates acides de soude, de potasse et de magnésium. Les matières organiques de l'urine comprennent notamment des matières colorantes, de l'urée, de l'acide urique et de la créatinine. L'urine est habituellement produite par l'organisme de vertébrés, préférentiellement des humains, dont elle permet d'évacuer les déchets. Il existe aussi des urines artificielles ou synthétiques, dont la composition est simplifiée par rapport à celle des urines naturelles, mais qui entrent aussi dans la définition du terme « urine » tel qu'utilisé ici. Les méthodes pour fabriquer de l'urine synthétique sont bien connues de l'homme du métier. La personne du métier pourra par exemple se référer au brevet EP 1 045 707 B1 ou aux articles de Shmaefsky, (Am. Biol. Teacher, 52 : 170-173; 1990 ; Am. Biol. Teacher, 57 : 428-430, 1995) et Degouy et al. (Toxicol In Vitro, 28(1) : 3-7, 2014). De façon préférée, l'urine synthétique ou artificielle telle qu'entendue dans la présente demande comprend de l'urée, de la créatinine, de l'acétate d'ammonium et de l'acide citrique. Ainsi, l'urine synthétique ou artificielle selon l'invention et la description comprend avantageusement de l'urée à une concentration comprise entre 9 et 24,5 g/L, du chlorure de sodium entre 3 et 14 g/L, de la créatinine 0,6 à 2,3 g/L, des sels d'ammonium, comme par exemple de l'acétate d'ammonium, entre 0,003 et 4,2 g/l et de l'acide citrique entre 0,09 et 0, 95 g/L.

Préférentiellement, le pH de l'urine synthétique selon l'invention et la description est compris entre 6 et 10 et plus préférentiellement entre 7 et 9 ; encore plus préférentiellement, le pH de l'urine synthétique selon l'invention et la description est égal à 8.

Par « effets délétères de l'urine», on entend ici les réactions pathologiques découlant de l'exposition de la peau à l'urine. Ces réactions pathologiques comprennent notamment l'érythème c'est-à-dire la rougeur, accompagné d'une sensation d'inconfort et de douleur, l'altération de la barrière et de l'épiderme, la formation de lésions et la desquamation.

Au sens des présents procédés, l'expression « l'efficacité d'une formulation dans la prévention ou la réduction des effets délétères de l'urine sur la peau d'enfant » signifie la capacité de la formulation à diminuer au moins l'un des effets délétères susmentionnés.

Dans un premier temps, la formulation ou l'actif d'intérêt est mis en contact avec une culture de peau reconstruite obtenue à partir d'un échantillon provenant d'un enfant. Cette mise en contact de l'actif d'intérêt avec le modèle de peau peut être faite directement. Alternativement, il pourra être avantageux de formuler l'actif d'intérêt, par exemple de façon à obtenir une composition liquide, afin de faciliter sa mise en contact avec le modèle de peau. Ainsi, par exemple, le procédé comprend en outre une étape de formulation de l'actif, notamment sous forme d'une solution liquide, en particulier aqueuse, préalablement à l'étape de mise en contact dudit actif avec un modèle de peau.

Les inventeurs ont précédemment montré que les profils d'expression de catégories spécifiques de gènes (par exemple, gènes de la barrière, de l'inflammation, de défense, des cellules souches) évolue en fonction de l'âge (demande PCT/EP2013/064926). L'homme du métier peut ainsi aisément caractériser la peau au niveau moléculaire depuis la naissance jusqu'à l'âge adulte. Plus particulièrement l'homme du métier notera que les cellules de la peau d'enfant présentent un profil d'expression spécifique des gènes impliqués dans des processus physiologiques particuliers, notamment le métabolisme cellulaire, la réponse au stress, l'inflammation, l'immunité, l'apoptose, la croissance/prolifération et le cycle cellulaire, la signalisation cellulaire, la migration et la différenciation, la barrière épidermique, l'adhésion et les cellules souches pluripotentes de la peau.

Au sens des présents procédés, le modèle de peau reconstruite obtenu à partir d'un échantillon cutané provenant d'un enfant peut être tout modèle tissulaire comprenant des cellules de la peau, notamment des kératinocytes, et dans lequel lesdites cellules cutanées ont été obtenues à partir d'un échantillon provenant d'un enfant.

Par « échantillon cutané », on entend ici tout échantillon contenant des cellules de la peau. Les échantillons cutanés selon l'invention et la description comprennent donc aussi bien les explants de peau frais obtenus directement du patient, que les cultures de cellules cutanées en suspension, les cultures de cellules cutanées en monocouche, les cultures de cellules cutanées en bicouche et les modèles tissulaires, dont les cultures de peaux reconstruites et les cultures de muqueuses reconstruites. Comme il est souvent difficile de travailler sur des explants frais, il est particulièrement avantageux, dans le cadre des présents procédés, d'utiliser des cultures de cellules cutanées. Avantageusement, les cellules cutanées comprennent des cellules normales, saines ou pathologiques, ou des cellules issues de lignées. Par exemple, les cellules cutanées mises en culture peuvent être des cellules obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans les présents procédés.

Alternativement, lesdites cellules cutanées peuvent être obtenues par différentiation de cellules souches (Guenou et al., Lancet, 374(9703) : 1745-1753, 2009 ; Nissan et al., Proc. Natl. Acad. Sci., 108(36) : 14861-14866, 2011 ; Kraehenbuehl et al., Nature Methods, 8 : 731-736, 2011).

Les cellules cutanées, qu'elles proviennent d'une biopsie ou qu'elles soient obtenues par différentiation de cellules souches, comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées comprennent au moins des kératinocytes et/ou des fibroblastes. De façon plus préférentielle, les cellules cutanées comprennent des kératinocytes et/ou des fibroblastes.

De nombreuses méthodes de culture de cellules cutanées sont connues de l'homme du métier. N'importe laquelle de ces méthodes peut être utilisée pour cultiver les cellules cutanées de l'invention et de la description. Avantageusement, les cellules cutanées sont cultivées et/ou conservées dans des conditions maintenant, au moins partiellement, un métabolisme cellulaire et/ou des fonctions cellulaires. La culture de cellules cutanées comprend donc aussi bien les cultures de cellules cutanées en suspension, les cultures de cellules cutanées en monocouche ou les cultures de cellules cutanées en bicouche que des modèles tissulaires, dont les cultures de peaux reconstruites et les cultures de muqueuses reconstruites.

Par exemple, les cultures de cellules cutanées en suspension sont pratiquées en routine dans un très grand nombre de laboratoires et ce, depuis plusieurs dizaines d'années. De même, les cultures de cellules cutanées en monocouche ou en bicouche sont connues et utilisées depuis très longtemps.

Par ailleurs, de nombreux modèles tissulaires, dont en particulier des modèles de peaux reconstruites et des modèles de muqueuses reconstruites (Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000; Schmalz et al., Eur J Oral Sci., 108(5) : 442-448, 2000 ; Black et al., Tissue Eng, 11(5-6) : 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2) : 107-113, 2009; Kinicoglu et al., Biomaterials, 32(25) : 5756-5764, 2011 ; Costin et al., Altern Lab Anim, 39(4) : 317-337, 2011 ; Auxenfans et al., J Tissue Eng Regen Med, 6(7) : 512-518, 2012 ; Lequeux et al., Skin Pharmacol Physiol, 25(1): 47-55, 2012; EP 29 678 ; EP 285 471 ; EP 789 074 ; EP 1 451 302 B1 ; EP 1 878 790 B1 ; EP 1 974 718 ; US 2007/0148,771 ; US 2010/0,099,576 ; WO 02/070729; WO 2006/063864 ; WO 2006/0,63865 ; WO 2007/064305) sont à la disposition de l'homme du métier et sont compris dans la portée des procédés.

Avantageusement, le modèle tissulaire comprend les modèles de peau reconstruite et les modèles de muqueuse reconstruite. Préférablement, le modèle de peau reconstruite est sélectionné dans le groupe comprenant les modèles de derme, contenant principalement des cellules stromales, et plus particulièrement encore des fibroblastes, les modèles d'épiderme constitué principalement de kératinocytes, les modèles d'hypoderme, les modèles de peau comprenant un derme et un épiderme, et les modèles de peau comprenant un derme, un épiderme et un hypoderme. Les modèles comprenant au moins un derme, forment des tissus de type conjonctifs, tandis que les modèles comprenant au moins un épiderme forment des épithélia stratifiés comprenant les couches caractéristiques du tissu considéré. Par exemple, on peut identifier dans les modèles d'épiderme une couche basale (*stratum basalis*), une couche épineuse (*stratum spinosum*), une couche granuleuse (*stratum granulosum*), et une couche cornée (*stratum corneum*). D'autre part, de façon préférée, le modèle de muqueuse reconstruite est un modèle de muqueuse de la bouche, de la gencive, du vagin ou de la cornée.

Avantageusement, ledit modèle est un modèle tissulaire de type conjonctif de matrice de derme comprenant un support matriciel de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, une membrane ou un film d'acide polyglycolique.

Dans ce groupe on trouve par exemple les modèles dermiques Skin$^2$™ modèle ZK1100 et Dermagraft® et Transcyte® (Advanced Tissue Sciences) ;

- un plastique traité culture cellulaire (formation d'un feuillet dermique : Michel et al., In Vitro Cell. Dev Biol.-Animal, 35 : 318-326, 1999) ;
- un gel ou une membrane à base d'acide hyaluronique (Hyalograft® 3D - Fidia Advanced Biopolymers) et/ou de collagène (comme par exemple un derme équivalent ou des lattices de collagène) et/ou de fibronectine et/ou de fibrine ; dans ce groupe on trouve par exemple le modèle dermique Vitrix® (Organogenesis) ;
- une matrice poreuse, surfacée ou non surfacée (par exemple un derme équivalent), réalisée à partir de collagène pouvant contenir un ou plusieurs glycosaminoglycanes et/ou éventuellement du chitosane (EP0296078A1, WO 01/911821 et WO 01/92322).

Dans ce groupe on trouve par exemple le modèle dermique Mimederm® (Coletica).

Ces supports matriciels comprennent des cellules stromales, en particulier des fibroblastes.

Avantageusement, ledit modèle de peau est un modèle d'épiderme comprenant un support matriciel de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable ;

dans ce groupe on trouve les modèles Epiderme reconstruits (Skinethic®) ainsi que le modèle EpiDerm®, (Mattek Corporation) ;

- un film ou une membrane à base d'acide hyaluronique et/ou de collagène et/ou de fibronectine et/ou de fibrine.

Dans ce groupe on peut citer particulièrement les modèles : Laserskin® (Fidia Advanced Biopolymers), Episkin® (L'Oréal).

Ces modèles peuvent être ensemencés par des fibroblastes dans la partie dermique.

Ces modèles, dans lesquels peuvent être intégrés ou non des fibroblastes, servent de support pour l'ensemencement des kératinocytes et la reconstitution de l'épiderme. Avantageusement, sont introduites, outre les kératinocytes, des cellules pigmentaires, des cellules immunocompétentes, des cellules nerveuses ; de préférence les cellules immunocompétentes sont des cellules de Langerhans.

Avantageusement, ledit modèle tissulaire est un modèle tissulaire de peau ou de muqueuse reconstruite comprenant un support matriciel dermique ou de chorion de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, ledit support inerte contenant ou non des cellules stromales, en particulier des fibroblastes,
- un gel à base de collagène et/ou acide hyaluronique et/ou fibronectine, et/ou de fibrine comprenant des cellules stromales en particulier des fibroblastes,
- une matrice poreuse, surfacée ou non surfacée, réalisée à partir de collagène pouvant contenir un ou plusieurs glycosaminoglycanes et/ou éventuellement du chitosane, ces matrices poreuses intégrant des cellules stromales, en particulier des fibroblastes,

- un derme desépidermisé ou derme mort, humain ou animal.

Dans ce groupe, on peut citer particulièrement les modèles Mimeskin (Coletica), EpidermFT™, EpiAirway™, EpiOccular™ EpiOral™, EpiGingival™, EpiVaginal™ (MatTek corporation), Human Corneal Epithelium (HCE), Human Oral Epithelium (HOE), Human Gingival Epithelium (HGE), Human Vaginal Epithelium (HVE) (Skinethic®), Phenion® Full Thickness Skin Model (Phenion) Apligraf® (Organogenesis), ATS-2000 (CellSystems® Biotechnologie Vertrieb) ainsi que Skin 2TM (ZK1200-1300-2000 Advanced Tissue Science).

Il existe par ailleurs des modèles dédiés à la thérapie tissulaire qui peuvent également être utilisés dans le cadre des présents procédés. On peut citer les modèles Epidex (Modex Thérapeutiques), Epibase® (Laboratoire Genévrier), Epicell™ (Genzyme), Autoderm™ et Transderm™ (Innogenetics).

Le support matriciel est ensuite ensemencé par des kératinocytes pour reconstruire l'épiderme et obtenir finalement une peau reconstruite.

Avantageusement, le modèle de peau utilisé comprend un modèle dans lequel a été incorporé au moins un type cellulaire complémentaire, comme des cellules endothéliales (CE) et/ou des cellules immunitaires telles que lymphocytes, macrophages, mastocytes, cellules dendritiques et/ou des cellules adipeuses et/ou des annexes cutanées, comme des poils, des cheveux, des glandes sébacées.

Après que la formulation d'intérêt et la culture de peau auront été mises en contact, le modèle de peau reconstruite ainsi traité pourra être exposé à l'urine.

Par « exposer le modèle de peau reconstruite à l'urine » on entend ici toute exposition ou mise en contact dudit modèle de peau reconstruite à une urine, que celle-ci soit naturelle ou synthétique.

Ainsi, de préférence, l'exposition dudit modèle de peau reconstruite à l'urine comprend ou consiste en l'exposition à l'urine naturelle, c'est-à-dire de l'urine produite naturellement par un être humain. De préférence, l'exposition du modèle de peau reconstruite à l'urine comprend ou consiste en l'exposition à une urine artificielle ou synthétique, c'est-à-dire une urine dont la composition est déterminée et constante. De préférence encore, l'exposition du modèle de peau reconstruite à l'urine comprend ou consiste en l'exposition à une urine synthétique comprenant de l'urée, de la créatinine, de l'acétate d'ammonium et de l'acide citrique. Préférentiellement, le pH de l'urine synthétique à laquelle est ainsi exposée la culture de peau est compris entre 6 et 10 et plus préférentiellement entre 7 et 9 ; encore plus préférentiellement, le pH de l'urine synthétique est égal à 8.

Après avoir exposé le modèle de peau reconstruite de l'invention et de la descirption à l'urine, l'homme du métier pourra procéder à la mesure du niveau d'expression des marqueurs biologiques de l'invention et de la description.

Par « marqueur biologique », on entend ici une caractéristique qui est objectivement mesurée et évaluée comme indicateur de processus biologiques normaux, de processus pathogéniques, ou de réponses pharmacologiques à une intervention thérapeutique. Un marqueur biologique désigne donc toute une gamme de substances et de paramètre divers. Par exemple, un marqueur biologique peut être une substance dont la détection indique un état pathologique particulier (par exemple la présence de la protéine C activée en tant que marqueur d'une infection), ou au contraire une substance dont la détection indique un état physiologique spécifique. Le marqueur biologique est préférentiellement un gène, les produits d'un gène tels que ses transcrits et les peptides issus de ses transcrits, un lipide, un sucre ou un métabolite.

Par exemple, le marqueur biologique est un gène, les produits d'un gène tels que des transcrits ou des peptides, un lipide, un sucre ou un métabolite dont les changements d'expression, en particulier de niveau d'expression, corrèlent avec un état physiologique de la peau d'enfant. Par exemple, le marqueur biologique est un peptide ayant une activité enzymatique.

L'homme du métier cherchant à déterminer à quelle classe appartient un marqueur génique ou protéique pourra facilement consulter la littérature scientifique pertinente ou se rapporter à des bases de données publiques telles que, par exemple, celles regroupées sur le site internet du National Center for Biotechnology Information (http://www.ncbi.nlm.nih.gov/guide/).

Les inventeurs ont particulièrement sélectionné des marqueurs dont la variation du niveau d'expression varie après exposition à l'urine de manière surprenante et inattendue chez les enfants. Les marqueurs sélectionnés présentent donc un intérêt particulier dans le cadre de la méthode de l'invention et de la description, dans la mesure où leur niveau d'expression est mesuré sur un modèle de peau reproduisant les caractéristiques de la peau d'enfant.

Les inventeurs ont ainsi montré que les marqueurs de l'inflammation sont particulièrement exprimés après que la peau d'enfant a été traitée à l'urine. L'inflammation est une réaction normale de défense de l'organisme, mais elle peut contribuer à la diminution de l'intégrité de la peau. En outre, les inventeurs ont montré que dans le même temps, l'expression des marqueurs de la barrière diminue. Enfin, les marqueurs des cellules souches sont eux-aussi affectés et par conséquent la capacité de renouvellement de l'épiderme. En revanche, la mise en contact de la peau avec une formulation active contre les effets de l'urine permet de prévenir et corriger les variations d'expression desdits marqueurs, ce qui souligne la pertinence de ceux-ci.

Le marqueur biologique est donc avantageusement un marqueur sélectionné dans le groupe des marqueurs de l'inflammation cutanée, de la fonction barrière et des marqueurs préférentiellement exprimés dans les cellules souches.

Par « marqueurs de l'inflammation cutanée », on entend ici les marqueurs dont la variation d'expression corrèle avec l'inflammation cutanée.

On entend par « inflammation » l'ensemble des mécanismes réactionnels de défense par lesquels l'organisme reconnaît, détruit et élimine toutes les substances qui lui sont étrangères. « L'inflammation cutanée » correspond plus particulièrement à une réaction du système immunitaire en réaction à une agression sur la peau, telle qu'une agression de l'environnement, occasionnant ou non une plaie, voire un dommage vasculaire le cas échéant. L'inflammation cutanée peut se manifester par un érythème, caractérisé par une rougeur associée à une vasodilatation locale, un oedème, caractérisée par un gonflement, et une sensation de chaleur. En outre, l'inflammation cutanée s'accompagne d'une variation de niveau d'expression ou de concentration de marqueur géniques ou protéiques bien connus de l'homme du métier, qui pourra se référer par exemple à Vahlquist (Acta Derm Venereol; 80: 161 ; 2000).

Le déclenchement et la poursuite de l'inflammation, sa diffusion à partir du foyer initial font appel à des facteurs qui sont synthétisés localement ou qui sont à l'état de précurseur inactif dans la circulation. Selon le type de médiateurs synthétisés, on peut différencier des processus particuliers dans la réaction d'inflammation. Ainsi, l'inflammation cutanée regroupe au moins trois processus distincts, l'inflammation protéique, l'inflammation lipidique et l'inflammation neurogène (ou neurogénique).

Par « inflammation protéique », on entend ici la production en réponse à une agression extérieure de médiateurs inflammatoires protéiques, tels que les cytokines IL-1, IL-2, IL-6, IL8 et TNFα, le système du complément, ou les protéines impliquées dans la coagulation, le cas échéant. Par « inflammation lipidique », on entend ici la production à ladite agression extérieure de médiateurs lipidiques, notamment les prostaglandines et les leucotriènes qui sont tous deux synthétisés à partir de l'acide arachidonique, ainsi que l'activation des enzymes responsables de cette production (Shimizu, Annu Rev Pharmacol Toxicol., 49: 123-150, 2009). Les médiateurs protéiques et lipidiques ainsi produits vont induire une cascade de réactions au sein de la peau impliquant d'autres cellules de l'inflammation, en particulier, des cellules immunitaires et vasculaires. Le résultat clinique se traduit notamment par des rougeurs, voire un oedème.

En réponse à une agression extérieure, le système neurosensoriel eut être stimulé et associé à la réaction inflammatoire mettant en oeuvre alors d'autres acteurs cellulaires comme les nerfs (ou terminaisons nerveuses) et des cellules telles que le mastocyte. Par « inflammation neurogène », on entend ici la libération par les terminaisons nerveuses, en réponse à une agression extérieure, de médiateurs spécifiques, notamment des neuropeptides (notamment les tachykinines dont la substance P, et le Calcitonin Gene-Related Peptide ou CGRP) ; participe aussi à l'inflammation neurogène, l'activité de récepteurs particuliers tels que la récepteur de la substance P ou le récepteur TRPV1. L'inflammation neurogène résulte le plus souvent en une sensation de de douleur et/ou d'inconfort et/ou de sensation de démangeaison (prurit).

Le marqueur de l'inflammation cutanée est de préférence choisi parmi les marqueurs de l'inflammation protéique, les marqueurs de l'inflammation lipidique et les marqueurs de l'inflammation neurogène.

Préférentiellement, le marqueur de l'inflammation protéique est choisi dans le groupe constitué par les interleukines, de préférence IL1α et IL8. L'interleukine IL1α humaine possède une séquence protéique représentée par la séquence de référence NCBI : NP_000566. Cette protéine est codée par le gène IL1A humain (référence NCBI : Gene ID: 3552), dont la séquence correspond à la référence NCBI : NM_000575. La séquence protéique de l'interleukine IL-8 humaine correspond à la séquence de référence NCBI : NP_000575. Cette protéine est codée par le gène IL8 humain (référence NCBI : Gene ID: 3576). La séquence de celui-ci est accessible sous la référence NCBI : NM_000584.

Le marqueur de l'inflammation lipidique est choisi avantageusement parmi les prostaglandines, dont en particulier la prostaglandine E2, et les enzymes de la synthèse de celles-ci à partir de l'acide arachidonique, en particulier PTGS2.

La prostaglandine E2 (PGE2), est un dérivé bien connu de l'acide arachidonique obtenu par l'action de la cyclo-oxygénase. Il existe deux isoformes de cyclo-oxygénases (COX) : la cyclo-oxygénase 1 qui est constitutive dans les tissus et la cyclo-oxygénase 2 qui est induite par les phénomènes inflammatoires. Une stimulation pro-inflammatoire (traumatisme, cytokines...) conduit ainsi à la synthèse de PGE 2 qui est responsable d'une vasodilatation (générant rougeur et oedème), d'une sensibilisation des nocicepteurs à la bradykinine et l'histamine (responsables de la douleur) et de la fièvre (avec les cytokines IL1 et IL6).

L'enzyme cyclo-oxygénase 2, aussi appelée prostaglandin-endoperoxide synthase (PTGS), est codée par le gène PTGS2 humain (référence NCBI : Gene ID: 5743). La séquence de ce gène est disponible sous la référence NCBI : NM_000963 et la séquence de la protéine sous la référence NCBI : NP_000954.

De façon préférée, le marqueur de l'inflammation neurogène est choisi parmi les neuropeptides et les récepteurs de neuropeptides, notamment les récepteurs TRPV1 et SRP. Le récepteur TRPV1 (Transient Receptor Potential Vanilloid 1) est une protéine membranaire de type canal cationique de la famille des TRP et de séquence de référence NCBI :

NP_061197. Au niveau de la peau, TRPV1 est exprimé par les kératinocytes, les mastocytes et les fibres nerveuses. En réponse à un agresseur, l'activation de TRPV1 conduit à la production de cytokines et de neuropeptides et est un acteur de l'inflammation neurogène. Le gène codant le récepteur TRPV1 est le gène TRPV1 (référence NCBI : Gene ID: 7442), dont la séquence a pour référence NCBI : NM_018727.

Le récepteur SPR (substance P receptor ; also known as neurokinin 1 receptor, NK1R, or tachykinin receptor 1, TACR1) est un récepteur couplé à la protéine G (GPCR) qui transmet le signal de la substance P (SP) et des autres tachykinines. Il est codé par le gène TACR1 humain dont la séquence a pour référence NCBI : NM_001058. Sa séquence peptidique est la séquence de référence NCBI : NP_001049.

Selon l'invention, le marqueur de l'inflammation cutanée est donc choisi parmi le groupe consistant en IL1$\alpha$, IL8, PTGS2, PGE2, TRPV1 et SPR.

Les présents inventeurs ont par ailleurs montré que l'urine induit une réduction de l'expression des marqueurs de la fonction barrière et des marqueurs préférentiellement exprimés dans les cellules souches.

Les « marqueurs de la barrière » selon l'invention et la description comprennent les marqueurs qui sont spécifiquement exprimés dans les couches de l'épiderme les plus externes et qui participent de la fonction barrière.

Comme il est bien connu de l'homme du métier, la peau a pour fonction principale d'établir une barrière de protection contre les atteintes de l'environnement tout en permettant certains échanges entre le milieu intérieur et le milieu extérieur. Cette fonction barrière est principalement portée par le *stratum corneum* de l'épiderme. Les lipides et les cornéodesmosomes intercellulaires ainsi que l'enveloppe cornée des cornéocytes en sont les constituants clés.

Cependant, sous le *stratum corneum,* les jonctions serrées constituent une seconde ligne de fonction barrière. Ces jonctions constituent au niveau du *stratum granulosum* une barrière de diffusion paracellulaire sélective prévenant de la pénétration de molécules délétères. Les jonctions serrées sont composées de différentes protéines transmembranaires comme notamment les claudines, l'occludine et ZO1.

Les fonctions de barrière portées par le *stratum corneum* et les jonctions serrées sont étroitement liées. En effet, l'altération de l'une ou l'autre peut influencer la formation de l'autre.

Préférentiellement, les marqueurs de la fonction barrière sont des marqueurs exprimés dans le *stratum corneum* ou des marqueurs exprimés dans les jonctions serrées du *stratum granulosum.* Dans l'invention, ledit marqueur de la barrière épidermique est sélectionné dans le groupe constitué de CNSDM (cornéodesmosine), IVL (involucrine), SMPD (shingomyélinase ou sphingomyéline diestérase), et CLDN1 (claudine 1).

Le cornéodesmosome est la seule structure jonctionnelle de la couche cornée, ce qui souligne l'importance de cette structure pour le maintien de l'intégrité de la couche cornée. Un des composants principaux du cornéodesmosome est la cornéodesmosine. Il s'agit de la seule protéine spécifique du cornéodesmosome : elle joue donc un rôle essentiel au sein de cette structure jonctionnelle. Sa séquence correspond à celle sous la référence NP_001255. Le gène CSDN (référence NCBI : Gene ID: 1041) code la cornéodesmosine et possède la séquence de référence NM_001264.

L'involucrine, de séquence NP_005538, et codée par le gène IVL (référence NCBI : Gene ID: 3713) qui a lui-même la séquence NM_005547, participe à la formation de l'enveloppe cornée des cornéocytes.

La shingomyélinase (SMase) ou sphingomyéline diestérase est une hydrolase impliquée dans le métabolisme des sphingolipides. Elle clive les sphingomyélines en phosphocholine et céramides 2 et 5, lesquels font partie de la matrice lipidique intercellulaire qui assure l'étanchéité de la couche cornée. La shingomyélinase est une protéine dont la séquence est représentée par la référence NCBI : NP_000534. Le gène codant cette enzyme est le gène SMPD (référence NCBI : Gene ID: 6609) de séquence correspondant à la référence NM_000543.

Les jonctions serrées représentent un des modes d'adhésion cellulaires, dans les tissus épithéliaux, Elles bloquent la circulation de fluides entre les cellules et assurent ainsi l'étanchéité entre deux compartiments tissulaires. Elles sont localisées à l'apex des cellules épithéliales où elles forment une bande continue tout autour qui assure l'étanchéité. Le gène CLDN 1 (référence NCBI : Gene ID: 9076) code la protéine claudine 1 qui est un est un des composants les plus importants des jonctions serrées. Cette protéine a une séquence correspondant à celle dont la référence NCBI est NP_066924. La séquence du gène CLDN1 est accessible sous la référence NM_021101.

Les « marqueurs préférentiellement exprimés dans les cellules souches » recouvrent les marqueurs, et plus spécifiquement les gènes et les protéines, qui sont spécifiquement présents dans les cellules souches épidermiques.

Par « cellule souche de l'épiderme » ou « cellule souche épidermique », on entend ici une cellule de l'épiderme capable de renouvellement à long terme. Les cellules souches épidermiques comprennent, entre autres, les cellules souches folliculaires, les cellules souches sébacées et les cellules souches basales, ces dernières étant aussi appelées cellules souches épidermiques interfolliculaires. On entend ici par « cellules souches folliculaires », « cellules souches sébacées » et « cellules souches basales » les cellules souches situées respectivement dans la région du bulge/renflement du follicule pileux, dans les glandes sébacées et dans la couche basale de l'épiderme. De préférence, les cellules souches épidermiques de l'invention sont des cellules souches basales.

Plus précisément, on entend par cellule souche épidermique une cellule dotée d'un potentiel élevé de renouvellement à long terme. Par « potentiel de renouvellement », on entend ici la capacité de subir au moins un cycle de division cellulaire. Un « potentiel élevé de renouvellement à long terme » représente donc la capacité d'une cellule d'entrer dans plusieurs cycles de division cellulaire successifs. Il est bien connu que les cellules différenciées de la peau ne sont pas capables d'effectuer plusieurs divisions successives (Fortunel et Martin, J Soc Biol, 202(1) : 55-65, 2008). Il est entendu ici que « successif » ne signifie pas « consécutif » et qu'il peut exister des périodes pendant lesquelles une cellule souche reste quiescente sans toutefois perdre son potentiel élevé de renouvellement à long terme.

La conservation d'un potentiel élevé de renouvellement à long terme s'exprime par une division asymétrique produisant deux cellules différentes. La première cellule fille est une cellule souche identique à la cellule souche mère, tandis que la seconde est une cellule d'amplification transitoire qui se divise de façon limitée sur une courte période puis entre dans le processus de différenciation. Avantageusement, les cellules souches épidermiques sont donc en outre capables de générer au moins un type de cellule épidermique par différenciation. Autrement dit, la cellule d'amplification transitoire est capable de donner au moins un type de cellule épidermique par différentiation. Préférentiellement, ladite cellule épidermique est un kératinocyte. Plus préférentiellement, la cellule d'amplification transitoire est capable de donner tous les types de cellules épidermiques par différentiation.

Préférentiellement, les marqueurs exprimés dans les cellules souches sont des marqueurs qui participent aux fonctions et à la protection des cellules souches. On citera par exemple les marqueurs ΔNp63, BIRC5 (survivine), FN1 (fibronectin 1), MCSP (Melanoma-associated Chondroitin Sulfate Proteoglycan), LRIG1 (Leucine-rich repeats and immunoglobulin-like domains protein 1), GJA1 (connexin 43), NID1 (nidogen 1), KRT15 (keratin 15), KRT19 (keratin 19), EGFR (epidermal growth factor receptor), CD71 (transferrin receptor), DSG3 (desmoglein 3), ITGB1BP1 (integrin beta1 binding protein), ITGA6 (integrin alpha 6), ITGB1 (integrin beta1) et ITGB4 (integrin beta 4) ou encore des marqueurs impliqués dans la signalisation et la régulation de l'activité des cellules souches comme Wnt/beta catenin, sonic hedgehog (SHH), NOTCH1 (Notch homolog 1, translocation-associated). Les marqueurs ΔNp63 et survivine sont des marqueurs de résistance à l'apoptose, ayant donc un rôle dans la survie des cellules souches. Les cytokératines 15 et 19 sont des marqueurs positifs des cellules souches, la cytokératine 15 étant un marqueur de survie de celles-ci. Le MCSP colocalise avec les intégrines dans les cellules qui ne se divisent pas, tandis que les intégrine beta1 (marqueur d'adhésion à la matrice extracellulaire de la membrane basale) et intégrine alpha 6 (constituant des hemidesmosomes marqueur de liaison des kératinocytes entre eux) sont des protéines de surface qui participent à la communication intercellulaire, régulent les processus de différenciation/prolifération, ainsi que l'interaction avec la niche. Le récepteur à la transférine CD71 est un marqueur de surface connu des cellules souches, qui est utilisé pour isoler, dans une population de cellules integrine-alpha6 positives, les cellules à haut pouvoir clonogéniques. Enfin, Lrig1 est un antagoniste du récepteur à l'Epidermal Growth Factor (EGFR), maintenant ainsi les cellules souches quiescentes, alors que le récepteur EGFR, qui est un marqueur dont l'absence caractérise les cellules souches, entraine au contraire les cellules dans la voie de la prolifération.

Le marqueur préférentiellement exprimé dans les cellules souches de l'invention est choisi dans le groupe constitué des marqueurs KRT19 (keratin 19), BIRC5 (survivine), LRIG1 (Leucine-rich repeats and immunoglobulin-like domains protein 1), ITGA6 (integrin alpha 6), ITGB1 (integrin beta1) et ITGB4 (integrin beta 4). Ces marqueurs sont bien connus de l'homme du métier. Les gènes KRT19 (référence NCBI : Gene ID: 3880), BIRC5 (référence NCBI : Gene ID: 332), LRIG1 (référence NCBI : Gene ID: 26018), ITGA6 (référence NCBI : Gene ID: 3655), ITGB1 (référence NCBI : Gene ID: 3688) et ITGB4 (référence NCBI : Gene ID: 3691) correspondent ainsi aux séquences représentées par les numéros d'accession Genbank suivants : NM_002276, NM_001012270, NM_015541, NM_000210, NM_002211 et NM_000213, respectivement. Les protéines keratin 19, survivine, Leucine-rich repeats and immunoglobulin-like domains protein 1, integrin alpha 6, integrin beta1 et integrin beta 4 correspondent quant à elles aux séquences représentées par les numéros d'accession Genbank suivants: NP_002267, NP_001012270, NP_056356, NP_000201, NP_002202 et NP_000204, respectivement.

Il sera par ailleurs évident à l'homme du métier que la méthode de l'invention et de la description permettra une évaluation de l'efficacité de la formulation ou de l'actif d'autant plus complète qu'un grand nombre de marqueurs de types différents seront utilisés.

Selon un mode de réalisation préféré, la méthode de l'invention et de la description comprend une étape c) de mesure du niveau d'expression d'une combinaison de marqueurs biologiques. Ladite combinaison selon l'invention et la description comprend ou consiste en :

- au moins un marqueur de l'inflammation cutanée et au moins un marqueur de la barrière tels que définis plus haut ; ou
- au moins un marqueur de l'inflammation cutanée et au moins un marqueur préférentiellement exprimé dans les cellules souches, tels que définis plus haut ; ou
- au moins un marqueur de la barrière et au moins un marqueur préférentiellement exprimé dans les cellules souches, tels que définis plus haut.

Dans un mode de réalisation plus préférentiel, ladite combinaison comprend au moins un marqueur de l'inflammation cutanée et au moins un marqueur de la barrière et au moins un marqueur préférentiellement exprimé dans les cellules souches, tels que définis plus haut.

L'utilisation de combinaisons de marqueurs comprenant au moins un marqueur de chacun des différents types indiqués plus haut est particulièrement avantageuse.

Pour chacun de ces marqueurs, le terme « niveau d'expression » se réfère à la concentration cellulaire dudit marqueur. Ainsi le niveau d'expression de la prostaglandine E2 correspond à la concentration dudit lipide dans la cellule. Si le marqueur est un gène, le « niveau d'expression » au sens de l'invention et de la description correspond à la concentration cellulaire d'au moins un des produits du gène dudit marqueur. Plus précisément, le niveau d'expression dudit marqueur biologique correspond à la quantité ou à la concentration cellulaire du transcrit dudit gène ou de la protéine issue dudit transcrit. De préférence, le niveau d'expression dudit marqueur biologique correspond à la quantité ou à la concentration cellulaire du transcrit dudit gène. Alternativement, le niveau d'expression dudit marqueur biologique correspond à la quantité ou à la concentration cellulaire de la protéine issue dudit transcrit.

Par la « mesure du niveau d'expression d'une combinaison de marqueurs biologiques », on entend ici la mesure du niveau d'expression de chacun des marqueurs de la combinaison. L'expression d'un gène peut être mesurée par exemple au niveau nucléotidique, en mesurant la quantité de transcrits dudit gène, et peut aussi être mesurée par exemple au niveau peptidique, en mesurant par exemple la quantité des protéines issus desdits transcrits. Ainsi, par « mesure du niveau d'expression dudit gène » on entend au sens de l'invention et de la description la mesure de la quantité de produit du gène sous sa forme peptidique ou sous sa forme nucléotidique.

De façon générale, l'expression du marqueur biologique sera détectée in vitro à partir du modèle de peau reconstruite.

En particulier, le procédé de l'invention et de la description peut comprendre une ou plusieurs étapes intermédiaires entre l'obtention du modèle de peau reconstruite et la mesure de l'expression du marqueur biologique, lesdites étapes correspondant à l'extraction à partir dudit modèle de peau reconstruite d'un échantillon lipidique, d'un échantillon d'ARNm (ou de l'ADNc correspondant) ou d'un échantillon de protéine.

Celui-ci peut ensuite être directement utilisé pour mesurer l'expression du marqueur. La préparation ou l'extraction d'ARNm (ainsi que la rétrotranscription de celui-ci en ADNc), de protéines ou de lipides à partir d'un échantillon cellulaire ne sont que des procédures de routine bien connues de l'homme du métier.

En ce qui concerne les médiateurs lipidiques tels que la prostaglandine E, il peut ne même pas être nécessaire de préparer un échantillon lipidique. En effet, ce médiateur est sécrété dans le milieu de culture. Il est alors aisé pour l'homme du métier de doser la prostaglandine E2 à partir dudit milieu de culture. Plusieurs méthodes pour doser et quantifier la prostaglandine E2 ont ainsi été décrites dans l'art, dont en particulier des méthodes ELISA. Une telle méthode est ainsi détaillée dans la partie expérimentale de la présente demande et l'homme du métier pourra se reporter à celle-ci. Il est en outre à noter que des kits sont disponibles commercialement pour le dosage de la prostaglandine E2 (par exemple, chez CissBio Assays ou chez Pierce).

Une fois qu'un échantillon d'ARNm (ou d'ADNc correspondant) ou de protéine est obtenu, l'expression du marqueur, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible.

Quand l'expression du marqueur est mesurée au niveau de l'ARNm (ou d'ADNc correspondant), n'importe quelle technologie habituellement utilisée par l'homme du métier peut être mise en oeuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose.

Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus..). Ces technologies sont utilisées en routine par l'homme du métier et il n'est donc pas besoin de les détailler ici.

Des exemples de mises en oeuvre des présents procédés basées sur l'analyse d'expression génique (puces à ADNc) et sur la PCR quantitative sont décrits dans la section expérimentale.

Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922; U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676-684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

Quand l'expression du marqueur est mesurée au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérométrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

Par « un niveau d'expression de référence d'un marqueur biologique », on entend ici tout niveau d'expression dudit marqueur utilisé à titre de référence. Par exemple, un niveau d'expression de référence peut être obtenu en mesurant le niveau d'expression du marqueur d'intérêt dans un modèle de peau d'enfant, dans des conditions particulières. L'homme du métier saura choisir ces conditions particulières en fonction de son objectif lors de la mise en oeuvre des procédés.

Par exemple, le niveau d'expression de référence d'un marqueur biologique est le niveau d'expression dudit marqueur obtenu dans un modèle de peau d'enfant non traité avec la formulation ou l'actif d'intérêt, et exposé aux UV.

Alternativement, le niveau d'expression de référence d'un marqueur biologique est le niveau d'expression dudit marqueur obtenu dans un modèle de peau d'enfant, mis en contact avec une formulation ou l'actif de référence, et exposé à l'urine.

Lorsque le niveau d'expression de référence est un niveau d'expression obtenu dans un modèle de peau exposé à l'urine, l'homme du métier comprendra aisément que les conditions d'expositions à l'urine du modèle de peau utilisé dans le procédé et du modèle utilisé pour obtenir un niveau d'expression de référence sont préférentiellement identiques. Ainsi, préférentiellement, la composition de l'urine artificielle utilisée, ainsi que la durée de l'exposition utilisée dans le procédé et du modèle utilisé pour obtenir un niveau d'expression de référence sont préférentiellement identiques.

Par exemple, l'homme du métier pourra utiliser comme formulation de référence toute formulation connue de l'art antérieur pour son effet dans la prévention des effets délétères de l'urine sur la peau.

Préférentiellement, la formulation de référence est choisie parmi une formule pour le change - émulsion phase huile continue, une formule pour le change - onguent phase grasse continue, une formule pour le change - pâte à l'eau, une formule pour le change - émulsion phase huile continue, un liniment pour le change et des lingettes pour le change. Encore plus préférentiellement, la formule pour le change - émulsion phase huile continue correspond à la formulation du Tableau 1, la formule pour le change - onguent phase grasse continue à celle du Tableau 2, la formule pour le change - pâte à l'eau à celle du Tableau 3, la formule pour le change - émulsion phase huile continue à celle du Tableau 4, le liniment pour le change à celle du Tableau 5 et les lingettes pour le change à celle du Tableau 6.

Tableau 1 : Formule P1 pour le change - émulsion phase huile continue

| MP | % |
|---|---|
| EAU | QSP |
| OXYDE DE ZINC | 5 à 20% |
| CAPRYLIC/CAPRIC TRIGLYCERIDE | 5 à 20% |
| COCO-CAPRYLATE/CAPRATE | 1 à 10% |
| POLYGLYCERYL-2-DIPOLYHYDROXYSTEARATE | 1 à 10% |
| GLYCERINE | 1 à 10% |
| CIRES | 1 à 5% |
| PERSEA GRATISSIMA OIL | 1 à 5% |
| POLYGLYCERYL-3 DIISOSTEARATE | 1 à 5% |
| MAGNESIUM SULFATE | 0 à 2% |
| STEARALKONIUM HECTORITE | 0 à 2% |
| CONSERVATEURS | 0 à 2% |
| PERSEA GRATISSIMA FRUIT EXTRACT / AVOCADO PERSEOSE | 0 à 5% |
| HELIANTHUS ANNUUS SEED OIL UNSAPONIFIABLES | 0 à 5% |
| UNDECYL DIMETHYL OXAZOLINE | 0 à 5% |
| ETHYL LINOLEATE | 0 à 5% |
| CAPRYLOYL GLYCINE | 0 à 5% |

Tableau 2 : Formule P2 pour le change - onguent phase grasse continue

| MP | % |
|---|---|
| OXYDE DE ZINC | 10 à 30% |
| HUILE DE FOIE DE POISSON / VITAMINE B5 | 10 à 30% |
| LANOLINE | 10 à 30% |
| VASELINE | QSP |
| ANTIOXYDANT | 0 à 2% |
| PARFUM / HUILE ESSENTIELLE | 0 à 2% |

Tableau 3 : Formule P3 pour le change - pâte à l'eau

| MP | % |
|---|---|
| VITAMINE B5 | 1 à 10% |
| OXYDE DE ZINC | 1 à 20% |
| DIOXYDE DE TITANE | 1 à 5% |
| CHARGE MINERALE (KAOLIN, TALC, SILICA) | 1 à 20% |
| ACTIF | 1 à 5% |
| CONSERVATEURS | 0 à 2% |
| EAU | QSP |
| GLYCERINE | 1 à 10% |

(suite)

| MP | % |
|---|---|
| SEQUESTRANT | 1 à 5% |

Tableau 4 : Formule pour le change - émulsion phase huile continue

| MP | % |
|---|---|
| VITAMINE B5 | 1 à 10% |
| ALCOOL CETYLIQUE | 10 à 30% |
| ALCOOL STEARYLIQUE | 10 à 30% |
| VASELINE / PARAFINE | 1 à 10% |
| ANTIOXYDANT | 0 à 2% |
| HUILE VEGETALE | 1 à 10% |
| CIRE | 1 à 10% |
| EAU | QSP |

Tableau 5: Liniment pour le change

| MP | % |
|---|---|
| HUILE VEGETALE | 20 à 50% |
| EAU + CALCIUM HYDROXYDE | QSP |
| STABILISANT | 1 à 5% |
| PERSEA GRATISSIMA FRUIT EXTRACT / AVOCADO PERSEOSE | 0 à 5% |
| HELIANTHUS ANNUUS SEED OIL UNSAPONIFIABLES | 0 à 5% |
| UNDECYL DIMETHYL OXAZOLINE | 0 à 5% |
| ETHYL LINOLEATE | 0 à 5% |
| CAPRYLOYL GLYCINE | 0 à 5% |
| CONSERVATEURS | 0 à 2% |
| ANTIOXYDANT | 0 à 2% |

Tableau 6 : Lingettes pour le change

| MP | % |
|---|---|
| EAU | QSP |
| GLYCERINE | 0 à 2% |
| PEG-40 HYDROGENATED CASTOR OIL | 0 à 2% |
| PARFUM | 0 à 2% |
| CONSERVATEURS | 0 à 2% |
| PERSEA GRATISSIMA FRUIT EXTRACT / AVOCADO PERSEOSE | 0 à 5% |
| HELIANTHUS ANNUUS SEED OIL UNSAPONIFIABLES | 0 à 5% |
| UNDECYL DIMETHYL OXAZOLINE | 0 à 5% |
| ETHYL LINOLEATE | 0 à 5% |

(suite)

| MP | % |
|---|---|
| CAPRYLOYL GLYCINE | 0 à 5% |
| AJUSTEUR pH | 0 à 1% |

Alternativement, le niveau d'expression de référence d'un marqueur biologique est le niveau d'expression dudit marqueur obtenu dans un modèle de peau obtenu à partir d'un échantillon cutané provenant d'un enfant, ledit modèle étant non traité avec la formulation ou l'actif d'intérêt, et non exposé à l'urine.

Alternativement, le niveau d'expression de référence d'un marqueur biologique est le niveau d'expression dudit marqueur obtenu dans un modèle de peau obtenu à partir d'un échantillon cutané provenant d'un enfant, ledit modèle étant non traité avec la formulation ou l'actif d'intérêt, mais exposé à l'urine.

Alternativement, le niveau d'expression de référence d'un marqueur biologique est le niveau d'expression dudit marqueur obtenu dans un modèle de peau obtenu à partir d'un échantillon cutané provenant d'un enfant, traité avec la formulation ou l'actif d'intérêt, et non exposé à l'urine.

L'homme du métier comprendra en outre aisément que la comparaison de l'étape d) est de préférence effectuée entre des mesures de niveaux d'expression obtenus pour des modèles de peau obtenus à partir d'échantillons cutanés provenant d'enfants, de structures histologiques similaires, voire identiques. Par « structures histologiques similaires », on entend au sens de la présente demande que les proportions relatives des types cellulaires compris dans les modèles de peau comparés sont similaires. Ainsi, il est préférable que les proportions relatives des types cellulaires compris dans le modèle de peau de l'étape a) ne diffèrent pas de plus de 5% des proportions relatives des types cellulaires compris dans le modèle de peau utilisé pour l'obtention du niveau d'expression de référence de l'étape d). Par « proportion relative d'un type cellulaire » on entend ici le rapport du nombre de cellules correspondant à ce type cellulaire sur le nombre de cellules totales comprises dans le modèle de peau. Ainsi, il est par exemple préférable que la proportion de kératinocytes sur le nombre de cellules totales dans le modèle de peau de l'étape a) ne diffère pas de plus de 5% de la proportion de kératinocytes sur le nombre de cellules totales dans le modèle de peau utilisé pour l'obtention du niveau d'expression de référence de l'étape d). Par « structures histologiques identiques », on entend ici que les proportions relatives des types cellulaires compris dans les modèles de peau comparés sont identiques. Au sens des présents procédés, les proportions relatives des types cellulaires compris dans le modèle de peau de mamelon de l'étape a) sont identiques aux proportions relatives des types cellulaires compris dans le modèle de peau utilisé pour l'obtention du niveau d'expression de référence de l'étape d) lorsqu'elles ne diffèrent pas de plus de 0,1%. Avantageusement, la proportion de kératinocytes sur le nombre de cellules totales dans le modèle de peau de l'étape a) ne diffère pas de plus de 0,1% de la proportion de kératinocytes sur le nombre de cellules totales dans le modèle de peau utilisé pour l'obtention du niveau d'expression de référence de l'étape d).

L'homme du métier comprendra tout aussi aisément que la comparaison de l'étape d) est de préférence effectuée entre des mesures de niveaux d'expression obtenus pour des modèles de peau qui sont de taille, de volume ou de poids similaires, voire identiques. Ainsi, il est préférable que la taille, le volume, ou le poids du modèle de peau de l'étape a) ne diffère pas de plus de 5% de la taille, du volume, ou du poids du modèle de peau utilisé pour l'obtention du niveau d'expression de référence de l'étape d). Plus préférentiellement, la taille, et le volume et le poids du modèle de peau de l'étape a) ne diffèrent pas de plus de 5% de la taille, du volume et du poids du modèle de peau utilisé pour l'obtention du niveau d'expression de référence de l'étape c). Encore plus préférentiellement, la taille, et le volume et le poids du modèle de peau de l'étape a) ne diffèrent pas de plus de 0,1% de la taille, du volume et du poids du modèle de peau utilisé pour l'obtention du niveau d'expression de référence de l'étape d).

Alternativement, si les modèles de peau diffèrent de plus de 5 % de par la taille, le volume et le poids, l'homme du métier pourra normaliser le niveau obtenu à l'étape c) et le niveau de référence de l'étape d) à l'aide d'un facteur de normalisation.

Ce facteur de normalisation pourra par exemple être un marqueur physique directement accessible tel que la masse de cellules de l'échantillon, ou la masse d'un constituant cellulaire, comme la masse d'ADN cellulaire ou la masse de protéines cellulaires.

Il peut aussi être avantageux d'utiliser comme facteur de normalisation le niveau d'expression d'un gène qui est exprimé au même niveau dans toutes les cellules, ou presque, de l'organisme. En d'autres termes, on utilise par exemple comme facteur de normalisation le niveau d'expression d'un gène de ménage. Selon un autre mode de réalisation, le niveau obtenu à l'étape c) et le niveau de référence de l'étape d) sont normalisés en utilisant le niveau d'expression, non pas des gènes de ménage, mais des protéines codées par ceux-ci. Un gène de ménage est un gène exprimé dans tous les types cellulaires, qui code une protéine ayant une fonction de base nécessaire pour la survie de tous les types cellulaires. Une liste de gènes de ménage humains peut être trouvée dans Eisenberg et al. (Trends in Genet, 19: 362-365, 2003). Les gènes de ménage incluent par exemple les gènes B2M, TFRC, YWHAZ, RPLO, 18S, GUSB, UBC, TBP, GAPDH, PPIA, POLR2A, ACTB, PGK1, HPRT1, IPO8 et HMBS.

L'homme du métier pourra ainsi aisément évaluer l'efficacité de la formulation d'intérêt en fonction de la comparaison de l'étape d).

Selon un autre aspect, il est ici décrit un kit pour la mise en oeuvre d'une méthode selon l'invention et la description, comprenant les moyens nécessaires à la mesure du niveau d'expression d'au moins un marqueur choisi parmi les marqueurs de l'inflammation cutanée, les marqueurs de la fonction barrière et les marqueurs préférentiellement exprimés dans les cellules souches. De préférence, le marqueur de l'inflammation cutanée est choisi parmi la prostaglandine E2, PTGS2, IL-1α, IL-8, TRPV1 et SPR, le marqueur de la fonction barrière est choisi parmi CNDSM, IVL, SMPD, et CLDN1, et le marqueur préférentiellement exprimé dans les cellules souches est choisi parmi KRT19, BIRC5, LRIG1, ITGA6, ITGB1 et ITGB4.

En particulier, le kit comprend en outre les moyens nécessaires à la mesure du niveau d'expression d'une combinaison de marqueurs biologiques choisie parmi le groupe comprenant ou consistant en :

- au moins un marqueur de l'inflammation cutanée et au moins un marqueur de la barrière tels que définis plus haut ; ou
- au moins un marqueur de l'inflammation cutanée et au moins un marqueur préférentiellement exprimé dans les cellules souches, tels que définis plus haut ; ou
- au moins un marqueur de la barrière et au moins un marqueur préférentiellement exprimé dans les cellules souches, tels que définis plus haut.

De préférence, ladite combinaison comprend au moins un marqueur de l'inflammation cutanée et au moins un marqueur de la barrière et au moins un marqueur préférentiellement exprimé dans les cellules souches, tels que définis plus haut.

Les exemples suivants sont fournis ici à titre d'illustration et ne sont pas, sauf indication contraire, destinés à être limitatifs.

EXEMPLES

**1. Effet de l'urine sur épidermes reconstruits d'un nourrisson de 1 an - Mise en place du modèle**

**1.1. Introduction**

L'érythème fessier ou dermite irritative du siège affecte plus de 35% des nourrissons avec un pic de fréquence autour de 10-12mois. Notre programme de recherche sur la peau de l'enfant, centré sur l'épiderme, a permis de mettre en évidence que :
La peau du nourrisson est plus fragile, elle remplit moins bien son rôle de barrière contre les agressions extérieures et les infections. La barrière étant en pleine construction, elle mobilise son capital en cellules souches (fortement présent à la naissance) le rendant ainsi plus vulnérable. Enfin, la peau du nourrisson possède un potentiel inflammatoire qui ne demande qu'à se développer.

Dans la zone du siège, sous les couches, l'atmosphère humide et l'occlusion font rapidement changer ces paramètres vers plus de fragilité, plus d'inflammation et une élévation du pH, ce qui crée un déséquilibre. De plus, dans ce contexte, le contact prolongé avec les urines et les selles (facteurs exogènes) peut provoquer rougeurs et douleurs, signes cliniques de l'érythème fessier.

Ainsi, associé aux facteurs exogènes connus, ces découvertes permettent de clarifier les mécanismes et facteurs déclencheurs de l'érythème fessier à savoir une altération de la barrière avec un capital en cellules souches sollicité et vulnérable et une réponse inflammatoire importante.

Pour aller plus loin dans la compréhension des mécanismes d'action et pour pouvoir disposer d'un modèle permettant d'évaluer les produits cosmétiques pour le change, nous avons développé un modèle mimant les effets délétères de l'urine sur des épidermes de nourrissons de 1 an (pic d'apparition de l'érythème fessier) et étudié les paramètres s'en trouvant modulés en analysant les marqueurs des différentes voies de l'inflammation, de la fonction barrière et des cellules souches.

Pour cela, des épidermes ont été reconstruits avec des kératinocytes issus d'un nourrisson de 1 an.

L'effet d'une préparation d'urine artificielle a été évalué sur ces épidermes en analysant la production d'un médiateur inflammatoire (ELISA) et l'expression génique (RT-qPCR) des marqueurs de l'inflammation, de la fonction barrière et des cellules souches.

**1.2. Matériel et Méthodes**

Les épidermes ont été réalisés avec les kératinocytes d'un donneur de 1 an (prélèvement préputial) selon le modèle dérivé de la méthode de Poumay et al (Arch Dermatol Res ; 296: 203-211, 2004). Après 2 jours de culture en immersion, les épidermes reconstruits ou RHE (Reconstructed Human Epidermis) ont été cultivés à l'interface air/liquide pendant 10 jours.

Pour chaque lot, à J10, les épidermes ont été incubés pendant 24h.

Après incubation, les épidermes ont été traités (urine) ou non (témoin) en topique avec une préparation d'urine artificielle (composée d'urée, créatinine, chlorure de sodium, acétate d'ammonium, acide citrique, pH 8) puis incubés pendant une nuit (16h).

Toutes les conditions expérimentales ont été réalisées en n=4.

Dosage du médiateur inflammatoire PGE2 dans les sous-nageants de culture

Après incubation, les quantités de PGE2 présentes dans les sous-nageants de culture ont été mesurées à l'aide d'un kit de dosage ELISA selon les spécifications du fournisseur.

Analyse de l'expression différentielle des gènes

L'expression des marqueurs a été évaluée par RT-qPCR sur les ARN messagers extraits des RHE de chaque traitement.

L'analyse de l'expression des gènes a été réalisée en n=2 à l'aide d'un PCR array contenant 30 gènes d'intérêt et 2 gènes de référence (housekeeping genes). Les ARN totaux de chaque échantillon ont été extraits à l'aide de TriPure Isolation Reagent® selon le protocole préconisé par le fournisseur. La quantité et la qualité des ARN ont été évaluées par électrophorèse capillaire (Bioanalyzer, Agilent). Les ADN complémentaires (ADNc) ont été synthétisés par transcription inverse des ARN en présence d'oligo(dT) et de l'enzyme « transcriptor Reverse Transcriptase ». L'ADNc obtenu a été quantifié par spectrophotométrie, puis les quantités d'ADNc ont été ajustées.

Les réactions de PCR ont été réalisées par PCR quantitative avec le système « light cycler » (Roche Molecular System Inc) et selon la procédure recommandée par le fournisseur. Le mélange réactionnel pour chaque échantillon a été : ADNc 10ng/$\mu$l, amorces des différents marqueurs utilisés, mélange réactionnel contenant l'enzyme taq DNA polymérase, le marqueur SYBR Green I (agent intercalant de l'ADN) et du MgCl2.

L'incorporation de fluorescence dans l'ADN amplifié est mesurée en continu au cours de cycles de PCR.

L'analyse quantitative des résultats est basée sur le recueil des cycles seuils (ou Ct pour threshold cycle). Le cycle seuil correspond au point où le signal d'émission de fluorescence est statistiquement et significativement plus élevé que le bruit de fond. Le cycle seuil est directement corrélé au nombre de copies initiales de l'ADN cible.

Pour chaque échantillon, le niveau d'expression du gène d'intérêt est normalisé par le niveau d'expression du gène de référence le plus stable, soit dans ces conditions expérimentales, le gène GAPDH.

Le tableau 7 liste les gènes qui ont été étudiés.

Ainsi est calculé $\Delta$Ct :

$$\Delta Ct = Ct_{\text{gène intérêt}} - Ct_{\text{gène de référence}}$$

Dans une deuxième étape, la variation, en fonction du traitement, du nombre de copies du gène d'intérêt a été déterminée et le $\Delta\Delta$Ct est calculé :

$$\Delta\Delta Ct = \Delta Ct_{\text{témoin}} - \Delta Ct_{\text{urine}}$$

Enfin, la quantité relative (QR) est calculée : QR = $(1+E)^{\Delta\Delta Ct}$.

Considérant que E (l'efficacité) est égale à 1, la quantité relative QR est donc :

$$QR = 2^{\Delta\Delta Ct}$$

Tableau 7 : Classification et nom des gènes

| Nom du cluster | Abréviation | Nom du gène |
|---|---|---|
| Housekeeping | RPS28 | Ribosomal protein 28S |
| | GADPH | Glyceraldehyde-3-phosphate dehydrogenase |
| Inflammation | IL1A | Interleukin 1, alpha |
| | IL8 | Interleukin 8 |
| | TRPV1 | Transient receptor potential vanilloid, member 1 |
| | TACR1 ou SPR | Tachykinin receptor 1 or Substance P receptor |
| | PTGS2 | Prostaglandin-endoperoxidase synthase 2 (prostaglandin G/H synthase and cyclooxygenase) |
| Différenciation épiderme, Fonction barrière | IVL | Involucrin |
| | CDSN | Corneodesmosin |
| | SMPD1 | Sphingomyelin phosphodiesterase 1, acid |
| | CLDN1 | Claudin 1 |
| Cellules souches | KRT19 | Keratin 19 |
| | ITGB1 | Integrin, beta 1 |
| | ITGB4 | Integrin, beta 4 |
| | ITGA6 | Integrin, alpha 6 |
| | LRIG1 | Leucin-rich repeats and immunoglobulin-like domains 1 |
| | BIRC5 | Baculoviral IAP repeat-containing 5 or survivin |

## 1.3. Résultats et Conclusions

### 1.3.1. Inflammation

La peau du nourrisson possède un potentiel inflammatoire exacerbé qui en contact d'agents « agresseurs » va se transformer en une réelle réponse inflammatoire source de rougeurs et de douleur. Dans ce contexte, l'exposition de la zone du siège aux urines (et aux selles) dans un environnement occlusif va générer une réaction inflammatoire impliquant 3 voies distinctes et interconnectées : l'inflammation protéique, l'inflammation lipidique et l'inflammation neurogène.

Au niveau de la peau, le kératinocyte est l'une des premières cellules participant à l'initiation de la réaction inflammatoire (inflammation précoce) en réponse à une agression de l'environnement.

Le kératinocyte « agressé » va alors produire des médiateurs inflammatoires protéiques (type cytokines IL1 et IL8) et/ou lipidiques (comme la PGE2 et les enzymes la générant à partir de l'acide arachidonique comme la PTGS2) qui vont induire une cascade de réactions au sein de la peau impliquant alors d'autres cellules de l'inflammation, immunitaires et vasculaires. Le résultat clinique se traduit par des rougeurs voir un oedème.

En réaction à une agression, le système neurosensoriel peut être stimulé et associé à la réaction inflammatoire mettant en oeuvre alors d'autres acteurs cellulaires comme les nerfs (ou terminaisons nerveuses) et des cellules telles que le mastocyte. Cette réaction inflammatoire qualifiée de neurogène implique aussi la production de médiateurs spécifiques pouvant être des neuropeptides (telle que la substance P) et aussi des récepteurs comme le récepteur à la substance P et le récepteur TRPV1*, notamment. Le résultat est de l'ordre du ressenti de douleurs et/ou d'inconfort et/ou de sensations de grattage (prurit).

*Le récepteur ou nocicepteur TRPV1 (Transient Receptor Potential Vanilloid 1) est une protéine membranaire de type canal cationique de la famille des TRP. Au niveau de la peau, TRPV1 est exprimé par les kératinocytes, les mastocytes et les fibres nerveuses. En réponse à un agresseur, l'activation de TRPV1 conduit à la production de cytokines et de neuropeptides et est un acteur de l'inflammation neurogène.

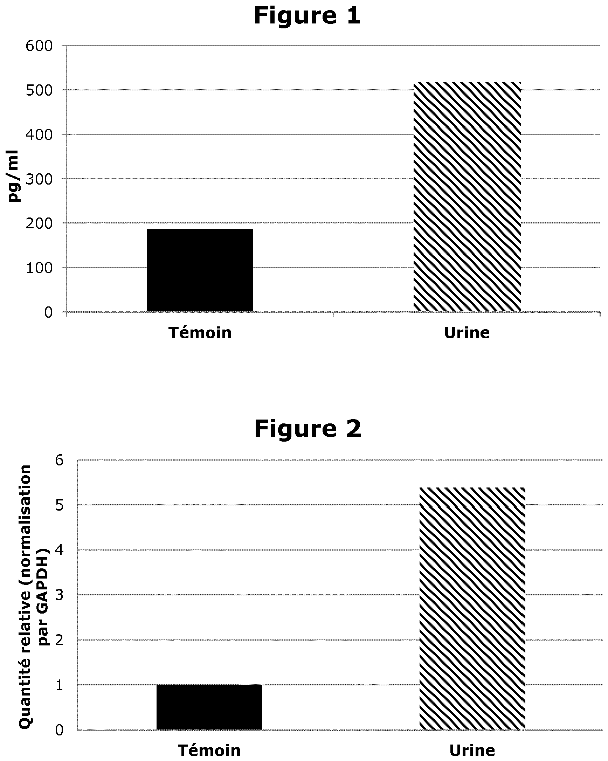

Le traitement des épidermes reconstruits avec la préparation d'urine artificielle a induit une production importante du médiateur lipidique de l'inflammation, PGE2 (Figure 1).

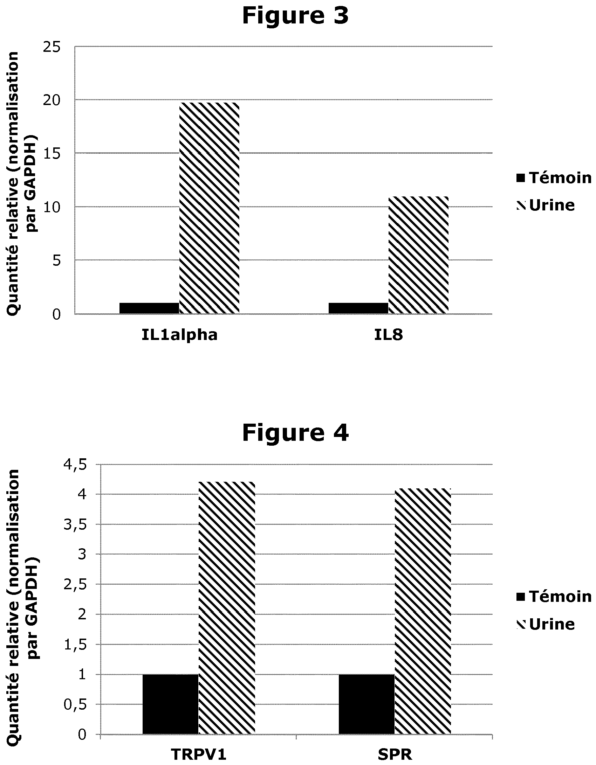

De plus, l'urine a induit la surexpression de gènes, marqueurs de l'inflammation lipidique (PTGS2, enzyme de la voie de synthèse des prostaglandines), de l'inflammation protéique (IL1 alpha et IL8) et de l'inflammation neurogène (récepteur à la substance P SPR et TRPV1) (Figures 2, 3 et 4 respectivement).

### 1.3.2. Fonction barrière

La couche la plus externe de l'épiderme, le *stratum corneum* (SC) constitue, grâce à sa structure particulière, une barrière protectrice contre les agressions de l'environnement. Le SC est le produit final de la différenciation de l'épiderme, où les kératinocytes sont transformés en cornéocytes ; ces cornéocytes étant enrobés d'une matrice lipidique intercellulaire constituée de céramides, de cholestérol et d'acides gras essentiels. Après extrusion des corps lamellaires, les précurseurs des lipides sont métabolisés en lipides matures. Dans le cas des précurseurs des céramides, ce sont les enzymes sphingomyélinase acide et beta glucocérébrosidase qui les transforment en céramides 2 et 5 (pour la sphingomyélinase) et en céramides 1, 3, 4, 6, 7, 8 et 9 (pour la glucocérébrosidase). Les cornéocytes sont entourés d'une enveloppe cornée protéique composée de nombreuses protéines comme l'involucrine, la loricrine, les small proline-rich protéines et la scielline notamment, solidement liées sous l'action des transglutaminases.

Les cornéodesmosomes sont des desmosomes modifiés, spécialisés qui attachent les cellules les unes aux autres dans le SC et assurent leur cohésion. Les corneodesmosomes sont composés de glycoprotéines transmembranaires telles que la desmogléine 1, desmocolline 1 ainsi que la cornéodesmosine.

La fonction de barrière de l'épiderme est principalement portée par le *stratum corneum* et les lipides et les cornéodesmosomes intercellulaires ainsi que l'enveloppe cornée des cornéocytes en sont les constituants clés.

Cependant, sous le SC, les jonctions serrées constituent une seconde ligne de fonction barrière. Elles forment au niveau du *stratum granulosum* des points d'ancrage entre les cellules formant une barrière physique au pôle apical des cellules. Ces jonctions constituent ainsi une barrière de diffusion paracellulaire sélective prévenant de la pénétration de molécules délétères. Les jonctions serrées sont composées de différentes protéines transmembranaires comme notamment les claudines, l'occludine et ZO1.

Les fonctions de barrière portées par le SC et les jonctions serrées sont étroitement liées. En effet, l'altération de l'une ou l'autre peut influencer la formation de l'autre.

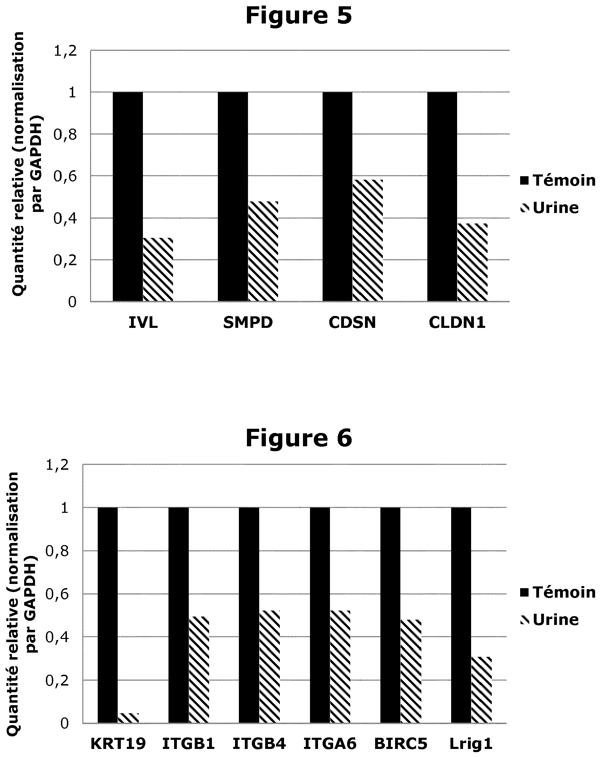

Dans nos conditions expérimentales, l'urine a induit la diminution de l'expression génique de marqueurs clés de la fonction barrière comme l'involucrine (enveloppe cornée), la sphyngomyélinase acide (maturation des céramides), la cornéodesmosine (cornéodesmosome) et la claudine 1 (jonction serrée) (Figure 5).

### 1.3.3. Cellules souches

Les cellules souches de tissus en renouvellement permanent sont classiquement définies comme étant des cellules rares et relativement quiescentes. Elles possèdent une capacité unique d'auto-renouvellement et de régénération tissulaire leur permettant d'assurer l'homéostasie et l'intégrité du tissu dans lequel elles résident.

Parmi les cellules souches de l'épiderme, les cellules souches interfolliculaires situées dans la couche basale constituent le réservoir épidermique principal en cellules souches. Ces cellules résident dans un microenvironnement anatomique et fonctionnel, la niche, qui contribue à maintenir leurs caractéristiques, notamment quand les conditions physiologiques changent. Les cellules souches interfolliculaires et leurs niches sont impliquées dans le maintien de l'intégrité et la régénération de l'épiderme.

Les cellules souches ne sont identifiables qu'en suivant plusieurs marqueurs :

- La kératine 19 est un marqueur de prolifération des cellules souches maintenant leurs caractéristiques.
- Les intégrines beta 1, beta 4 et alpha 6 (récepteurs de surface cellulaire) sont identifiées comme des marqueurs des cellules souches, et plus particulièrement de l'interaction avec leur niche. Elles contribuent également à réguler les processus de différenciation et de prolifération
- La survivine (BIRC5) est un marqueur des cellules souches régulant le cycle cellulaire. Marqueur de résistance à l'apoptose, elle a un rôle dans la survie des cellules souches face à des agressions (comme les UV).
- Lrig1 est un antagoniste du récepteur à l'Epidermal Growth Factor et un marqueur maintenant les cellules souches quiescentes.

Le traitement avec l'urine artificielle a fortement limité l'expression de plusieurs des marqueurs caractéristiques des cellules souches et de leur niche (Figure 6).

Le traitement avec une préparation d'urine artificielle des épidermes reconstruits avec des cellules de peau de nourrissons de 1 an a permis de montrer, dans ces conditions expérimentales, que l'urine a un effet délétère sur les marqueurs de la fonction barrière et sur les marqueurs des cellules souches. De plus l'urine a induit une inflammation importante impliquant différentes voies, d'une part l'inflammation non spécifique avec différents marqueurs protéiques et lipidiques et d'autre part l'inflammation neurogène.

Une fois validé, ce modèle a été utilisé pour évaluer l'efficacité de produits cosmétiques formulés pour une application au moment du change sur la zone du siège des nourrissons.

**2. Evaluation de l'efficacité de produits cosmétiques pour le change sur le modèle d'épidermes reconstruits de nourrissons de 1 an « agressés » avec de l'urine artificielle**

**2.1. Matériel et Méthodes**

Les épidermes ont été reconstruits de la même façon que décrit plus haut.

Les épidermes reconstruits issus de kératinocytes de peau de nourrisson de 1 an ont été traités ou non en topique par les produits à l'essai et incubés pendant 24h. Après incubation, les épidermes ont à nouveau été traités ou non en topique par les produits à l'essai puis ont été exposés à l'urine artificielle (appliquée également en topique). Les RHE ont ensuite été incubés pendant une nuit (16h). Toutes les conditions expérimentales ont été réalisées en n=4.

Quatre produits cosmétiques pour le change, de formulations distinctes, ont été testés et nommés P1, P2, P3 et P4.

Ces produits sont décrits dans les Tableaux 1 à 4, respectivement.

Le dosage du médiateur inflammatoire PGE2 dans les sous-nageants de culture et l'analyse de l'expression différentielle des gènes ont été réalisés comme décrit précédemment.

Pour chaque échantillon, le niveau d'expression du gène d'intérêt est normalisé par le niveau d'expression du gène de référence le plus stable, soit dans ces conditions expérimentales, le gène GAPDH. Le calcul des quantités relatives a été réalisé comme décrit précédemment.

Après calcul des quantités relatives des gènes d'intérêt dans les différentes conditions, Témoin (sans urine sans produit à l'essai), Urine (sans produit à l'essai) et Produit P (Produit à l'essai + urine), le pourcentage de modulation (stimulation ou inhibition) exercé par le produit à l'essai sur l'effet délétère de l'urine est calculé selon les formules ci-dessous :

$$\text{Stimulation (\%)} = (QR_{\text{produit à l'essai}} - QR_{\text{urine}}) \times 100$$

$$\text{Inhibition (\%)} = (QR_{\text{urine à l'essai}} - QR_{\text{produit}}) \times 100$$

Analyse statistique pour calculer la significativité des résultats : les comparaisons intergroupes ont été réalisées à l'aide du test T de Student bilatéral non apparié ; *p<0,05 ; **p<0,01 ; *** p<0,001.

**2.2. Résultats et Conclusion**

2.2.1. Inflammation

Tableau 8 : % inhibition de l'expression des gènes IL1A et IL8 par les produits à l'essai (par rapport à l'urine)

| Produit à l'essai | IL1A | IL8 | Moy Inhibition Infl. Protéique IL1 et IL8 | Significativité |
|---|---|---|---|---|
| P1 | 43 | 29 | 36 | * |
| P2 | 24 | 15 | 20 | |
| P3 | 32 | 30 | 31 | * |
| P4 | 39 | 0 | 20 | |

Seuls les produits P1 et P3 inhibent l'IL1A et l'IL8.

Tableau 9 : % inhibition de l'expression du gène PTGS2 et de la production de PGE2 par les produits à l'essai (par rapport à l'urine)

| Produit à l'essai | PGE2 | PTGS2 | Moy. Infl. Lipidique PGE2 et PTGS2 | Significativité |
|---|---|---|---|---|
| P1 | 75 | 88 | 82 | ** |
| P2 | 103 | 74 | 89 | *** |
| P3 | 28 | 76 | 52 | * |
| P4 | 58 | 75 | 67 | * |

Tous les produits inhibent les marqueurs lipidiques. P2 et P1 sont les plus performants.

Tableau 10 : % inhibition de l'expression des gènes TRPV1 et SPR par les produits à l'essai (par rapport à l'urine)

| Produit à l'essai | TRPV1 | SPR | Moy. Infl. Neurogène | Significativité |
|---|---|---|---|---|
| P1 | 89 | 51 | 70 | *** |
| P2 | 65 | 23 | 44 | ** |
| P3 | 72 | 0 | 36 | |
| P4 | 93 | 30 | 62 | ** |

Tous les produits, sauf P3, inhibent efficacement les marqueurs de l'inflammation neurogène, P1 étant le plus efficace.

Tableau 11 : % inhibition de l'inflammation (moyenne des % pour tous les marqueurs inflammatoires)

| Produit à l'essai | % Inhibition Inflammation (total) | Significativité | RANG |
|---|---|---|---|
| P1 | 63 | ** | 1 |
| P2 | 51 | * | 2 |
| P3 | 40 | | 4 |
| P4 | 49 | * | 3 |

Cette analyse des marqueurs de l'inflammation permet de montrer d'une part l'efficacité plus ou moins importante des produits à l'essai à moduler l'inflammation induite par l'urine et d'autre part de classer les produits selon leur efficacité.

P1 > P2 > P4 > P3. Le produit P1 est le seul produit capable d'inhiber tous les marqueurs de l'inflammation quel qu'ils soient de façon significative

2.2.2. Barrière

Tableau 12 : % stimulation des marqueurs de la barrière par les produits à l'essai (par rapport à l'urine)

| Produit à l'essai | IVL | Significativité | CDS N | Significativité | SMP D1 | Significativité | CLD N1 | Significativité |
|---|---|---|---|---|---|---|---|---|
| P1 | 123 | ** | 64 | *** | 106 | *** | 41 | ** |
| P2 | 226 | *** | 32 | * | 51 | ** | 23 | * |

(suite)

| Produit à l'essai | IVL | Significativité | CDS N | Significativité | SMP D1 | Significativité | CLD N1 | Significativité |
|---|---|---|---|---|---|---|---|---|
| P3 | 74 | * | -14 | | 115 | *** | -21 | |
| P4 | 80 | * | 31 | | 90 | *** | 2 | |

Les produits P1 et P2 sont performants pour stimuler l'expression des différents marqueurs de la barrière, et P1 stimule tous les marqueurs de façon plus importante, hormis la stimulation de l'involucrine plus importante par P2. Les produits P3 et P4 n'ont pas d'effet sur les marqueurs cornéodesmosine et claudine 1.

Tableau 13 : % stimulation des marqueurs de la barrière (moyenne des % pour tous les marqueurs barrière)

| Produit à l'essai | % Protection/Renfort Fonction Barrière | Significativité | RANG |
|---|---|---|---|
| P1 | 84 | *** | 1 |
| P2 | 83 | ** | 2 |
| P3 | 39 | * | 4 |
| P4 | 51 | * | 3 |

Cette analyse des marqueurs de la barrière permet de montrer d'une part l'efficacité plus ou moins importante des produits à l'essai à renforcer et protéger la barrière altérée par l'urine et d'autre part de classer les produits selon leur efficacité.

P1 > P2 > P4 ≥ P3. Le produit P1 est le produit le plus efficace pour renforcer et protéger la barrière épidermique.

2.2.3. Cellules souches

Tableau 14 : % stimulation des marqueurs des cellules souches par les produits à l'essai (par rapport à l'urine)

| Produit à l'essai | KRT 19 | ITGB1 | ITGA6 | ITGB4 | BIRC5 | Lrig1 |
|---|---|---|---|---|---|---|
| P1 | 93 *** | 62 ** | 45 * | 90 *** | 48 ** | 91 ** |
| P2 | 51 * | 83 *** | 77 * | 66 ** | 14 | 27 * |
| P3 | 116 *** | 33 | 20 | -6 | 48 ** | 32 * |
| P4 | 80 *** | 78 * | 36 * | -43 | 42 ** | 73 * |

Pour identifier les cellules souches interfolliculaires dans l'épiderme il est important de suivre en parallèle plusieurs marqueurs spécifiques des cellules souches et de leur niche. Les produits à l'essai stimulent plus ou moins l'expression des marqueurs KRT19, ITGB1, A6 et B4, BIRC5 et Lrig1 altérés par l'urine. P1 stimule de façon significative tous les marqueurs des cellules souches. P2 et P4 stimulent 5 des 6 marqueurs des cellules souches. P3 n'agit que sur 3 des marqueurs.

Tableau 15 : % stimulation des marqueurs des cellules souches  (moyenne des % pour tous les marqueurs cellules souches)

| Produit à l'essai | % Protection cellules souches | Significativité | RANG |
|---|---|---|---|
| P1 | 71 | ** | 1 |
| P2 | 53 | * | 2 |
| P3 | 40 | * | 4 |
| P4 | 44 | * | 3 |

Cette analyse des marqueurs des cellules souches permet de montrer d'une part l'efficacité plus ou moins importante des produits à l'essai à protéger les cellules souches altérées par l'urine et d'autre part de classer les produits selon leur efficacité.

P1 > P2 > P4 ≥ P3. Le produit P1 est le produit le plus efficace pour protéger les cellules souches de la couche basale de l'épiderme.

**Revendications**

1. Procédé d'évaluation de l'efficacité *in vitro* d'un actif ou d'une formulation sur la prévention des effets délétères de l'urine sur la peau d'enfant, **caractérisé en ce que** ledit procédé comprend les étapes suivantes:

   a) mettre en contact ledit actif ou ladite formulation avec un modèle de peau reconstruite, ledit modèle étant obtenu à partir d'un échantillon cutané provenant d'un enfant ;
   b) exposer à l'urine le modèle de peau reconstruite de l'étape a) ;
   c) mesurer le niveau d'expression d'au moins un marqueur biologique dans le modèle de peau de l'étape b) ; et
   d) évaluer l'efficacité dudit actif ou de ladite formulation en fonction du niveau de l'étape c) ;

   dans lequel ledit marqueur est choisi parmi :

   - les marqueurs de l'inflammation cutanée, ledit marqueur de l'inflammation cutanée étant choisi parmi la prostaglandine E2, PTGS2, IL-1$\alpha$, IL-8, TRPV1 et SPR ;
   - les marqueurs de la fonction barrière, ledit marqueur de la fonction barrière étant choisi parmi CNDSM, IVL, SMPD, et CLDN1 ; et
   - les marqueurs préférentiellement exprimés dans les cellules souches, ledit marqueur préférentiellement exprimé dans les cellules souches étant choisi parmi KRT19, BIRC5, LRIG1, ITGA6, ITGB1 et ITGB4.

2. Procédé d'évaluation de l'efficacité *in vitro* d'un actif ou d'une formulation dans la réduction des effets délétères de l'urine sur la peau d'enfant, **caractérisé en ce que** ledit procédé comprend les étapes suivantes:

   a) exposer à l'urine un modèle de peau reconstruite, ledit modèle étant obtenu à partir d'un échantillon cutané provenant d'un enfant ;
   b) mettre en contact ledit actif ou ladite formulation avec le modèle de peau reconstruite de l'étape a) ;
   c) mesurer le niveau d'expression d'au moins un marqueur biologique dans le modèle de peau de l'étape b) ; et
   d) évaluer l'efficacité dudit actif ou de ladite formulation en fonction du niveau de l'étape c) ;

dans lequel ledit marqueur est choisi parmi :

- les marqueurs de l'inflammation cutanée, ledit marqueur de l'inflammation cutanée étant choisi parmi la prostaglandine E2, PTGS2, IL-1$\alpha$, IL-8, TRPV1 et SPR ;
- les marqueurs de la fonction barrière, ledit marqueur de la fonction barrière étant choisi parmi CNDSM, IVL, SMPD, et CLDN1 ; et
- les marqueurs préférentiellement exprimés dans les cellules souches, ledit marqueur préférentiellement exprimé dans les cellules souches étant choisi parmi KRT19, BIRC5, LRIG1, ITGA6, ITGB1 et ITGB4.

3. Procédé d'identification d'un actif ou d'une formulation pour la prévention des effets délétères de l'urine sur la peau d'enfant, **caractérisé en ce que** ledit procédé comprend les étapes suivantes:

a) mettre en contact un actif ou une formulation candidat avec un modèle de peau reconstruite, ledit modèle étant obtenu à partir d'un échantillon cutané provenant d'un enfant ;
b) exposer à l'urine le modèle de peau de l'étape a) ;
c) mesurer le niveau d'expression d'au moins un marqueur biologique dans le modèle de peau de l'étape b) ; et
d) déterminer si ledit actif ou ladite formulation candidat est une formulation ou un actif pour la prévention des effets délétères de l'urine sur la peau d'enfant en fonction du niveau de l'étape c) ;

dans lequel ledit marqueur est choisi parmi :

- les marqueurs de l'inflammation cutanée, ledit marqueur de l'inflammation cutanée étant choisi parmi la prostaglandine E2, PTGS2, IL-1$\alpha$, IL-8, TRPV1 et SPR ;
- les marqueurs de la fonction barrière, ledit marqueur de la fonction barrière étant choisi parmi CNDSM, IVL, SMPD, et CLDN1 ; et
- les marqueurs préférentiellement exprimés dans les cellules souches, ledit marqueur préférentiellement exprimé dans les cellules souches étant choisi parmi KRT19, BIRC5, LRIG1, ITGA6, ITGB1 et ITGB4.

4. Procédé d'identification d'un actif ou d'une formulation pour la réduction des effets délétères de l'urine sur la peau d'enfant, **caractérisé en ce que** ledit procédé comprend les étapes suivantes:

a) exposer à l'urine un modèle de peau reconstruite, ledit modèle étant obtenu à partir d'un échantillon cutané provenant d'un enfant ;
b) mettre en contact un actif ou une formulation candidat avec le modèle de peau de l'étape a) ;
c) mesurer le niveau d'expression d'au moins un marqueur biologique dans le modèle de peau de l'étape b) ; et
d) déterminer si ledit actif ou ladite formulation candidat est une formulation ou un actif pour la réduction des effets délétères de l'urine sur la peau d'enfant en fonction du niveau de l'étape c) ;

dans lequel ledit marqueur est choisi parmi :

- les marqueurs de l'inflammation cutanée, ledit marqueur de l'inflammation cutanée étant choisi parmi la prostaglandine E2, PTGS2, IL-1$\alpha$, IL-8, TRPV1 et SPR ;
- les marqueurs de la fonction barrière, ledit marqueur de la fonction barrière étant choisi parmi CNDSM, IVL, SMPD, et CLDN1 ; et
- les marqueurs préférentiellement exprimés dans les cellules souches, ledit marqueur préférentiellement exprimé dans les cellules souches étant choisi parmi KRT19, BIRC5, LRIG1, ITGA6, ITGB1 et ITGB4.

5. Procédé d'évaluation de la tolérance d'un actif ou d'une formulation sur la peau d'enfant exposée à l'urine, ledit procédé comprenant les étapes suivantes :

a) mettre en contact ledit actif ou ladite formulation avec un modèle de peau reconstruite, ledit modèle étant obtenu à partir d'un échantillon cutané provenant d'un enfant ;
b) exposer à l'urine en présence de l'actif ou de la formulation le modèle de peau reconstruite de l'étape a) ;
c) mesurer le niveau d'expression d'au moins un marqueur biologique dans le modèle de peau de l'étape b) ; et
d) déterminer si ledit agent actif, l'actif ou de la formulation est bien toléré par la peau d'enfant en fonction du niveau de l'étape c) ;

dans lequel ledit marqueur est choisi parmi :

- les marqueurs de l'inflammation cutanée, ledit marqueur de l'inflammation cutanée étant choisi parmi la prostaglandine E2, PTGS2, IL-1α, IL-8, TRPV1 et SPR ;
- les marqueurs de la fonction barrière, ledit marqueur de la fonction barrière étant choisi parmi CNDSM, IVL, SMPD, et CLDN1 ; et
- les marqueurs préférentiellement exprimés dans les cellules souches, ledit marqueur préférentiellement exprimé dans les cellules souches étant choisi parmi KRT19, BIRC5, LRIG1, ITGA6, ITGB1 et ITGB4.

6. Procédé selon l'une quelconque des revendications 1 à 5, **caractérisé en ce que** l'étape c) comprend la mesure d'une combinaison de marqueurs biologiques, ladite combinaison comprenant :

   • au moins un marqueur de l'inflammation cutanée et au moins un marqueur de la barrière ; ou
   • au moins un marqueur de l'inflammation cutanée et au moins un marqueur préférentiellement exprimé dans les cellules souches ; ou
   • au moins un marqueur de la barrière et au moins un marqueur préférentiellement exprimé dans les cellules souches.

7. Procédé selon la revendication 6, **caractérisé en ce que** ladite combinaison comprend au moins un marqueur de l'inflammation cutanée et au moins un marqueur de la barrière et au moins un marqueur préférentiellement exprimé dans les cellules souches.

8. Procédé selon l'une quelconque des revendications 1 à 7, **caractérisé en ce que** l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

9. Procédé selon l'une quelconque des revendications 1 à 8, **caractérisé en ce que** le modèle de peau reconstruite est choisi parmi les cultures de cellules cutanées en suspension, les cultures de cellules cutanées en monocouche, les cultures de cellules cutanées en bicouche, les cultures de peaux reconstruites et les cultures de muqueuses reconstruites.

10. Procédé selon la revendication 9, **caractérisé en ce que** les cellules dudit modèle proviennent d'un explant de tissu cutané ou de cellules souches différenciées en cellules cutanées.

11. Procédé selon l'une quelconque des revendications 8 ou 9, **caractérisé en ce que** ledit modèle comprend au moins des fibroblastes ou des kératinocytes.

**Patentansprüche**

1. Verfahren zur Bewertung der Wirksamkeit *in vitro* eines Wirkstoffs oder einer Formulierung zur Vorbeugung gegen die schädlichen Wirkungen des Urins auf die Haut von Kindern, **dadurch gekennzeichnet, dass** das Verfahren die folgenden Schritte umfasst:

   a) Inkontaktversetzen des Wirkstoffs oder der Formulierung mit einem Modell rekonstruierter Haut, wobei das Modell aus einer Hautprobe eines Kindes erhalten wurde;
   b) Aussetzen dem Urin des Modells rekonstruierter Haut von Schritt a);
   c) Messen des Expressionsniveaus mindestens eines biologischen Markers in dem Hautmodell von Schritt b); und
   d) Beurteilen der Wirksamkeit des Wirkstoffs oder der Formulierung in Abhängigkeit des Niveaus von Schritt c);

   wobei der Marker ausgewählt ist aus:

   - den Markern der Hautentzündung, wobei der Marker der Hautentzündung aus dem Prostaglandin E2, PTGS2, IL-1α, IL-8, TRPV1 und SPR ausgewählt ist;
   - den Markern der Barrierefunktion, wobei der Marker der Barrierefunktion aus CNDSM, IVL, SMPD und CLDN1 ausgewählt ist; und
   - den Markern, die vorzugsweise in den Stammzellen exprimiert sind, wobei der Marker vorzugsweise in den Stammzellen exprimiert ist, die aus KRT19, BIRC5, LRIG1, ITGA6, ITGB1 und ITGB4 ausgewählt sind.

**2.** Verfahren zur Bewertung der Wirksamkeit *in vitro* eines Wirkstoffs oder einer Formulierung zur Reduzierung der schädlichen Wirkungen des Urins auf die Haut von Kindern, **dadurch gekennzeichnet, dass** das Verfahren die folgenden Schritte umfasst:

a) Aussetzen dem Urin eines Modells rekonstruierter Haut, wobei das Modell aus einer Hautprobe eines Kindes erhalten wurde;
b) Inkontaktversetzen des Wirkstoffs oder der Formulierung mit dem Modell rekonstruierter Haut von Schritt a);
c) Messen des Expressionsniveaus mindestens eines biologischen Markers in dem Hautmodell von Schritt b); und
d) Beurteilen der Wirksamkeit des Wirkstoffs oder der Formulierung in Abhängigkeit des Niveaus von Schritt c);

wobei der Marker ausgewählt ist aus:

- den Markern der Hautentzündung, wobei der Marker der Hautentzündung aus dem Prostaglandin E2, PTGS2, IL-1$\alpha$, IL-8, TRPV1 und SPR ausgewählt ist;
- den Markern der Barrierefunktion, wobei der Marker der Barrierefunktion aus CNDSM, IVL, SMPD und CLDN1 ausgewählt ist; und
- den Markern, die vorzugsweise in den Stammzellen exprimiert sind, wobei der Marker vorzugsweise in den Stammzellen exprimiert ist, die aus KRT19, BIRC5, LRIG1, ITGA6, ITGB1 und ITGB4 ausgewählt sind.

**3.** Verfahren zur Identifizierung eines Wirkstoffs oder einer Formulierung für die Vorbeugung gegen die schädlichen Wirkungen des Urins auf die Haut von Kindern, **dadurch gekennzeichnet, dass** das Verfahren die folgenden Schritte umfasst:

a) Inkontaktversetzen eines Kandidaten-Wirkstoffs oder -Formulierung mit einem Modell rekonstruierter Haut, wobei das Modell aus einer Hautprobe eines Kindes erhalten wurde;
b) Aussetzen dem Urin des Hautmodells von Schritt a) ;
c) Messen des Expressionsniveaus mindestens eines biologischen Markers in dem Hautmodell von Schritt b); und
d) Bestimmen, ob der Kandidaten-Wirkstoff oder - Formulierung eine Formulierung oder ein Wirkstoff zur Vorbeugung gegen die schädlichen Wirkungen des Urins auf die Haut von Kindern in Abhängigkeit des Niveaus von Schritt c) ist;

wobei der Marker ausgewählt ist aus:

- den Markern der Hautentzündung, wobei der Marker der Hautentzündung aus dem Prostaglandin E2, PTGS2, IL-1$\alpha$, IL-8, TRPV1 und SPR ausgewählt ist;
- den Markern der Barrierefunktion, wobei der Marker der Barrierefunktion aus CNDSM, IVL, SMPD und CLDN1 ausgewählt ist; und
- den Markern, die vorzugsweise in den Stammzellen exprimiert sind, wobei der Marker vorzugsweise in den Stammzellen exprimiert ist, die aus KRT19, BIRC5, LRIG1, ITGA6, ITGB1 und ITGB4 ausgewählt sind.

**4.** Verfahren zur Identifizierung eines Wirkstoffs oder einer Formulierung zur Reduzierung der schädlichen Wirkungen des Urins auf die Haut von Kindern, **dadurch gekennzeichnet, dass** das Verfahren die folgenden Schritte umfasst:

a) Aussetzen dem Urin eines Modells rekonstruierter Haut, wobei das Modell aus einer Hautprobe eines Kindes erhalten wurde;
b) Inkontaktversetzen eines Kandidaten-Wirkstoffs oder -Formulierung mit dem Hautmodell von Schritt a);
c) Messen des Expressionsniveaus mindestens eines biologischen Markers in dem Hautmodell von Schritt b); und
d) Bestimmen, ob der Kandidaten-Wirkstoff oder - Formulierung eine Formulierung oder ein Wirkstoff zur Reduzierung der schädlichen Wirkungen des Urins auf die Haut von Kindern in Abhängigkeit des Niveaus von Schritt c) ist;

wobei der Marker ausgewählt ist aus:

- den Markern der Hautentzündung, wobei der Marker der Hautentzündung aus dem Prostaglandin E2, PTGS2, IL-1$\alpha$, IL-8, TRPV1 und SPR ausgewählt ist;

- den Markern der Barrierefunktion, wobei der Marker der Barrierefunktion aus CNDSM, IVL, SMPD und CLDN1 ausgewählt ist; und
- den Markern, die vorzugsweise in den Stammzellen exprimiert sind, wobei der Marker vorzugsweise in den Stammzellen exprimiert ist, die aus KRT19, BIRC5, LRIG1, ITGA6, ITGB1 und ITGB4 ausgewählt sind.

**5.** Verfahren zur Beurteilung der Verträglichkeit eines Wirkstoffs oder einer Formulierung auf die Haut von Kindern, die dem Urin ausgesetzt ist, wobei das Verfahren die folgenden Schritte umfasst:

a) Inkontaktversetzen des Wirkstoffs oder der Formulierung mit einem Modell rekonstruierter Haut, wobei das Modell aus einer Hautprobe eines Kindes erhalten wurde;
b) Aussetzen dem Urin bei Anwesenheit des Wirkstoffs oder der Formulierung des Modells rekonstruierter Haut von Schritt a);
c) Messen des Expressionsniveaus mindestens eines biologischen Markers in dem Hautmodell von Schritt b); und
d) Bestimmen, ob das Wirkmittel, der Wirkstoff oder die Formulierung von der Haut des Kindes gut vertragen wird in Abhängigkeit des Niveaus von Schritt c);

wobei der Marker ausgewählt ist aus:

- den Markern der Hautentzündung, wobei der Marker der Hautentzündung aus dem Prostaglandin E2, PTGS2, IL-1$\alpha$, IL-8, TRPV1 und SPR ausgewählt ist;
- den Markern der Barrierefunktion, wobei der Marker der Barrierefunktion aus CNDSM, IVL, SMPD und CLDN1 ausgewählt ist; und
- den Markern, die vorzugsweise in den Stammzellen exprimiert sind, wobei der Marker vorzugsweise in den Stammzellen exprimiert ist, die aus KRT19, BIRC5, LRIG1, ITGA6, ITGB1 und ITGB4 ausgewählt sind.

**6.** Verfahren nach einem der Ansprüche 1 bis 5, **dadurch gekennzeichnet, dass** der Schritt c) das Messen einer Kombination biologischer Marker umfasst, wobei die Kombination umfasst:

• mindestens einen Marker der Hautentzündung und mindestens einen Marker der Barriere; oder
• mindestens einen Marker der Hautentzündung und mindestens einen Marker, der vorzugsweise in den Stammzellen exprimiert ist; oder
• mindestens einen Marker der Barriere und mindestens einen Marker, der vorzugsweise in den Stammzellen exprimiert ist.

**7.** Verfahren nach Anspruch 6, **dadurch gekennzeichnet, dass** die Kombination mindestens einen Marker der Hautentzündung und mindestens einen Marker der Barriere und mindestens einen Marker, der vorzugsweise in den Stammzellen exprimiert ist, umfasst.

**8.** Verfahren nach einem der Ansprüche 1 bis 7, **dadurch gekennzeichnet, dass** die Hautprobe von einem Spender kommt, der aus der Gruppe ausgewählt ist, die von den Neugeborenen, deren Alter zwischen 0 und 1 Monat liegt, den Säuglingen, deren Alter zwischen 1 Monat und 2 Jahren liegt, und den Kindern, deren Alter zwischen 2 Jahren und 16 Jahren liegt, gebildet ist.

**9.** Verfahren nach einem der Ansprüche 1 bis 8, **dadurch gekennzeichnet, dass** das Modell rekonstruierter Haut aus den Hautzellkulturen in Suspension, den Hautzellkulturen in Monolage, den Hautzellkulturen in Doppellage, den Kulturen rekonstruierter Haut und den Kulturen rekonstruierter Schleimhaut ausgewählt ist.

**10.** Verfahren nach Anspruch 9, **dadurch gekennzeichnet, dass** die Zellen des Modells aus einem Explantat von Hautgewebe oder von in Hautzellen differenzierten Stammzellen stammen.

**11.** Verfahren nach einem der Ansprüche 8 oder 9, **dadurch gekennzeichnet, dass** das Modell mindestens Fibroblasten oder Keratinozyten umfasst.

**Claims**

**1.** A method for evaluating the *in vitro* efficacy of an active agent or a formulation in preventing the harmful effects of urine on children's skin, **characterized in that** said method comprises the following steps:

a) contacting said active agent or said formulation with a reconstructed skin model, said model being obtained from a skin sample from a child;
b) exposing the reconstructed skin model of step a) to urine;
c) measuring the expression level of at least one biological marker in the skin model of step b); and
d) evaluating the efficacy of said active agent or said formulation as a function of the level of step c);

wherein said marker is selected from:

- the skin inflammation markers, said skin inflammation marker being selected from prostaglandin E2, PTGS2, IL-1$\alpha$, IL-8, TRPV1 and SPR;
- the barrier function markers, said barrier function marker being selected from CNDSM, IVL, SMPD and CLDN1; and
- the markers preferentially expressed in stem cells, said marker preferentially expressed in stem cells being selected from KRT19, BIRC5, LRIG1, ITGA6, ITGB1 and ITGB4.

2. A method for evaluating the *in vitro* efficacy of an active agent or a formulation in reducing the harmful effects of urine on children's skin, **characterized in that** said method comprises the following steps:

a) exposing a reconstructed skin model to urine, said model being obtained from a skin sample from a child;
b) contacting said active agent or said formulation with the reconstructed skin model of step a);
c) measuring the expression level of at least one biological marker in the skin model of step b); and
d) evaluating the efficacy of said active agent or said formulation as a function of the level of step c);

wherein said marker is selected from:

- the skin inflammation markers, said skin inflammation marker being selected from prostaglandin E2, PTGS2, IL-1$\alpha$, IL-8, TRPV1 and SPR;
- the barrier function markers, said barrier function marker is selected from CNDSM, IVL, SMPD and CLDN1; and
- the markers preferentially expressed in stem cells, said marker preferentially expressed in stem cells being selected from KRT19, BIRC5, LRIG1, ITGA6, ITGB1 and ITGB4.

3. A method for identifying an active agent or a formulation for preventing the harmful effects of urine on children's skin, **characterized in that** said method comprises the following steps:

a) contacting a candidate active agent or formulation with a reconstructed skin model, said model being obtained from a skin sample from a child;
b) exposing the skin model of step a) to urine;
c) measuring the expression level of at least one biological marker in the skin model of step b); and
d) determining whether said candidate active agent or formulation is a formulation or an active agent for preventing the harmful effects of urine on children's skin as a function of the level of step c);

wherein said marker is selected from:

- the skin inflammation markers, said skin inflammation marker being selected from prostaglandin E2, PTGS2, IL-1$\alpha$, IL-8, TRPV1 and SPR;
- the barrier function markers, said barrier function marker being selected from CNDSM, IVL, SMPD and CLDN1; and
- the markers preferentially expressed in stem cells, said marker preferentially expressed in stem cells being selected from KRT19, BIRC5, LRIG1, ITGA6, ITGB1 and ITGB4.

4. A method for identifying an active agent or a formulation for reducing the harmful effects of urine on children's skin, **characterized in that** said method comprises the following steps:

a) exposing a reconstructed skin model to urine, said model being obtained from a skin sample from a child;
b) contacting a candidate active agent or formulation with the skin model of step a);
c) measuring the expression level of at least one biological marker in the skin model of step b); and

d) determining whether said candidate active agent or formulation is a formulation or an active agent for reducing the harmful effects of urine on children's skin as a function of the level of step c);

wherein said marker is selected from:

- the skin inflammation markers, said skin inflammation marker being selected from prostaglandin E2, PTGS2, IL-1$\alpha$, IL-8, TRPV1 and SPR;
- the barrier function markers, said barrier function marker being selected from CNDSM, IVL, SMPD and CLDN1; and
- the markers preferentially expressed in stem cells, said marker preferentially expressed in stem cells being selected from KRT19, BIRC5, LRIG1, ITGA6, ITGB1 and ITGB4.

5. A method for evaluating the tolerance of an active agent or a formulation on children's skin exposed to urine, said method comprising the following steps:

a) contacting said active agent or said formulation with a reconstructed skin model, said model being obtained from a skin sample from a child;
b) exposing the reconstructed skin model of step a) to urine in the presence of the active agent or the formulation;
c) measuring the expression level of at least one biological marker in the skin model of step b); and
d) determining whether said active agent or said formulation is well tolerated by children's skin as a function of the level of step c);

wherein said marker is selected from:

- the skin inflammation markers, said skin inflammation marker being selected from prostaglandin E2, PTGS2, IL-1$\alpha$, IL-8, TRPV1 and SPR;
- the barrier function markers, said barrier function marker being selected from CNDSM, IVL, SMPD and CLDN1; and
- the markers preferentially expressed in stem cells, said marker preferentially expressed in stem cells being selected from KRT19, BIRC5, LRIG1, ITGA6, ITGB1 and ITGB4.

6. The method of any one of claims 1 to 5, **characterized in that** step c) comprises the measurement of a combination of biological markers, said combination comprising:

• at least one skin inflammation marker and at least one barrier marker; or
• at least one skin inflammation marker and at least one marker preferentially expressed in stem cells; or
• at least one barrier marker and at least one marker preferentially expressed in stem cells.

7. The method of claim 6, **characterized in that** said combination comprises at least one skin inflammation marker and at least one barrier marker and at least one marker preferentially expressed in stem cells.

8. The method of any one of claims 1 to 7, **characterized in that** the skin sample comes from a donor selected from the group consisting of newborns, between 0 and 1 month of age, infants, between 1 month and 2 years of age, and children between 2 years and 16 years of age.

9. The method of any one of claims 1 to 8, **characterized in that** the reconstructed skin model is selected from suspended skin cell cultures, monolayer skin cell cultures, bilayer skin cell cultures, reconstructed skin cultures and reconstructed mucosal cultures.

10. The method of claim 9, **characterized in that** the cells of said model come from a skin tissue explant or from stem cells differentiated into skin cells.

11. The method of any one of claims 8 or 9, **characterized in that** said model comprises at least fibroblasts or keratinocytes.

# Figure 1

# Figure 2

# Figure 3

# Figure 4

## Figure 5

## Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1045707 B1
- EP 2013064926 W
- EP 29678 A
- EP 285471 A
- EP 789074 A
- EP 1451302 B1
- EP 1878790 B1
- EP 1974718 A
- US 20070148771 A
- US 20100099576 A
- WO 02070729 A
- WO 2006063864 A
- WO 2006063865 A
- WO 2007064305 A
- EP 0296078 A1

- WO 01911821 A
- WO 0192322 A
- US 4882127 A
- US 4849077 A
- US 7556922 B
- US 6723513 B
- WO 03066896 A
- WO 2007111924 A
- US 20080020392 A
- WO 2006084132 A
- US 20090186349 A
- US 20090181860 A
- US 20090181385 A
- US 20060275782 A
- EP 1141399 B1

**Littérature non-brevet citée dans la description**

- **HOGAN et al.** *J Allergy,* 2012, vol. 2012, 901940
- **CHIOU et al.** *Skin Pharmacol Physiol,* 2004, vol. 17, 57-66
- **NIKOLOVSKI et al.** *J Invest Dermatol,* 2008, vol. 128, 1728-1736
- **STAMATAS et al.** *Pediatr Dermatol,* 2010, vol. 27, 125-131
- **TELOFSKI et al.** *Dermatol Res Pract,* 2012, vol. 2012, 198789
- **FLUHR et al.** *Br J Dermatol,* 2012, vol. 166 (3), 483-90
- **FLUHR et al.** *Br J Dermatol,* 2014
- **DEGOUY et al.** *Toxicol In Vitro,* 2014, vol. 28 (1), 3-7
- **FLUHR et al.** *Exp Dermatol.,* 2010, vol. 19 (6), 483-492
- **FLUHR et al.** *Br J Dermatol.,* 2014
- **MOSMAN et al.** *J Immunol Methods,* 1983, vol. 65 (1-2), 55-63
- **SHMAEFSKY.** *Am. Biol. Teacher,* 1990, vol. 52, 170-173
- *Am. Biol. Teacher,* 1995, vol. 57, 428-430
- **GUENOU et al.** *Lancet,* 2009, vol. 374 (9703), 1745-1753
- **NISSAN et al.** *Proc. Natl. Acad. Sci.,* 2011, vol. 108 (36), 14861-14866
- **KRAEHENBUEHL et al.** *Nature Methods,* 2011, vol. 8, 731-736

- **ROSDY et al.** *In Vitro Toxicol.,* 1997, vol. 10 (1), 39-47
- **PONEC et al.** *J Invest Dermatol.,* 1997, vol. 109 (3), 348-355
- **PONEC et al.** *Int J Pharm.,* 2000, vol. 203 (1-2), 211-225
- **SCHMALZ et al.** *Eur J Oral Sci.,* 2000, vol. 108 (5), 442-448
- **BLACK et al.** *Tissue Eng,* 2005, vol. 11 (5-6), 723-733
- **DONGARI-BATGTZOGLOU ; KASHLEVA.** *Nat Protoc,* 2006, vol. 1 (4), 2012-2018
- **BECHTOILLE et al.** *Tissue Eng,* 2007, vol. 13 (11), 2667-2679
- **VRANA et al.** *Invest Ophthalmol Vis Sci,* 2008, vol. 49 (12), 5325-5331
- **KINICOGLU et al.** *Biomaterials,* 2009, vol. 30 (32), 6418-6425
- **AUXENFANS et al.** *Eur J Dermatol,* 2009, vol. 19 (2), 107-113
- **KINICOGLU et al.** *Biomaterials,* 2011, vol. 32 (25), 5756-5764
- **COSTIN et al.** *Altern Lab Anim,* 2011, vol. 39 (4), 317-337
- **AUXENFANS et al.** *J Tissue Eng Regen Med,* 2012, vol. 6 (7), 512-518
- **LEQUEUX et al.** *Skin Pharmacol Physiol,* 2012, vol. 25 (1), 47-55
- **MICHEL et al.** *In Vitro Cell. Dev Biol.-Animal,* 1999, vol. 35, 318-326

- **VAHLQUIST.** *Acta Derm Venereol,* 2000, vol. 80, 161
- **SHIMIZU.** *Annu Rev Pharmacol Toxicol.,* 2009, vol. 49, 123-150
- **FORTUNEL ; MARTIN.** *J Soc Biol,* 2008, vol. 202 (1), 55-65
- **SHENDURE ; JI.** *Nat Biotechnol,* 2008, vol. 26 (10), 1135-45
- **PIHLAK et al.** *Nat Biotechnol,* 2008, vol. 26 (6), 676-684
- **FULLER et al.** *Nature Biotechnol,* 2009, vol. 27 (11), 1013-1023
- **MARDIS.** *Genome Med.,* 2009, vol. 1 (4), 40
- **METZKER.** *Nature Rev. Genet.,* 2010, vol. 11 (1), 31-46
- **EISENBERG et al.** *Trends in Genet,* 2003, vol. 19, 362-365
- **POUMAY et al.** *Arch Dermatol Res,* 2004, vol. 296, 203-211